# EUROPEAN PATENT SPECIFICATION

(11) **EP 1 605 965 B1**
(45) Date of publication and mention of the grant of the patent: **26.12.2012**
(21) Application number: 04739073.7
(22) Date of filing: 26.03.2004
(51) Int. Cl.: A61K 38/18, C12N 5/07, A01K 67/00, C07K 14/475, G01N 33/68

(54) **USE OF SAPOSIN-RELATED PROTEINS FOR PREVENTING AND TREATING OBESITY, DIABETES AND/OR METABOLIC SYNDROME**
VERWENDUNG VON MIT SAPOSIN VERWANDTEN PROTEINEN ZUR PRÄVENTION UND BEHANDLUNG VON ADIPOSITAS, DIABETES UND/ODER DES METABOLISCHEN SYNDROMS
UTILISATION DE PROTEINES ASSOCIEES A LA SAPOSINE POUR LA PREVENTION ET LE TRAITEMENT DE L'OBESITE, DU DIABETE ET/OU DU SYNDROME METABOLIQUE

(30) Priority: 26.03.2003 EP 03006948
(43) Date of publication of application: 21.12.2005
(73) Proprietor: DeveloGen Aktiengesellschaft, 37079 Göttingen (DE)
(72) Inventor: ONICHTCHOUK, Daria, 79100 Freiburg (DE); EULENBERG, Karsten, 79639 Grenzach / Wyhlen (DE); NGUYEN, Tri, 79576 Weil am Rhein (DE); BURK, Ulrike, 37073 Göttingen (DE)
(74) Representative: Weiss, Wolfgang
(86) International application number: PCT/EP2004/003244
(87) International publication number: WO 2004/084930

(56) References cited:
- EP-A- 1 072 609
- WO-A-01/55198
- WO-A-87/02037
- WO-A1-00/45837
- WO-A1-00/62862
- WO-A1-01/23528
- US-A- 4 918 161
- US-A1- 2001 024 824
- US-B1- 6 271 196
- US-B1- 6 500 431

## Description

This invention relates to the use of saposin-related proteins, to the use of polynucleotides encoding these, in the diagnosis, study, prevention, and treatment of obesity and/or diabetes mellitus and/or metabolic syndrome.

Many human proteins serve as pharmaceutically active compounds. Several classes of human proteins that serve as such active compounds include hormones, cytokines, cell growth factors, and cell differentiation factors. Most proteins that can be used as a pharmaceutically active compound fall within the family of secreted proteins. Secreted proteins are generally produced within cells at rough endoplasmic reticulum, are then exported to the golgi complex, and then move to secretory vesicles or granules, where they are secreted to the exterior of the cell via exocytosis. Examples for commercially used secreted proteins are human insulin, thrombolytic agents, interferons, interleukins, colony stimulating factors, human growth hormone, transforming growth factor beta, tissue plasminogen activator, erythropoeitin, and various other proteins. Receptors of secreted proteins, which are membrane-bound proteins, also have potential as therapeutic or diagnostic agents.

It is, therefore, important for developing new pharmaceutical compounds to identify secreted proteins that can be tested for activity in a variety of animal models. Thus, in light of the pervasive role of secreted proteins in human physiology, a need exists for identifying and characterizing novel functions for human secreted proteins and the genes that encode them. This knowledge will allow one to detect, to treat, and to prevent medical diseases, disorders, and/or conditions by using secreted proteins or the genes that encode them.

Obesity is one of the most prevalent metabolic disorders in the world. It is still a poorly understood human disease that becomes as a major health problem more and more relevant for western society. Obesity is defined as a body weight more than 20% in excess of the ideal body weight, frequently resulting in a significant impairment of health. Obesity may be measured by body mass index, an indicator of adiposity or flatness. Further parameters for defining obesity are waist circumferences, skinfold thickness and bioimpedance. It is associated with an increased risk for cardiovascular disease, hypertension, diabetes mellitus type II, hyperlipidaemia and an increased mortality rate.

Obesity is influenced by genetic, metabolic, biochemical, psychological, and behavioral factors and can be caused by different reasons such as non-insulin dependent diabetes, increase in triglycerides, increase in carbohydrate bound energy and low energy expenditure. As such, it is a complex disorder that must be addressed on several fronts to achieve lasting positive clinical outcome. Since obesity is not to be considered as a single disorder but as a heterogeneous group of conditions with (potential) multiple causes, it is also characterized by elevated fasting plasma insulin and an exaggerated insulin response to oral glucose intake (Koltermann J., (1980) Clin. Invest 65, 1272-1284). A clear involvement of obesity in type 2 diabetes mellitus can be confirmed (Kopelman P.G., (2000) Nature 404, 635-643).

Triglycerides and glycogen are used as the body's fuel energy storage. Glycogen is a large branched polymer of glucose residues that is mainly stored in liver and muscle cells. Glycogen synthesis and degradation is central to the control of the blood glucose level. Triglycerides are stored in the cytoplasm of adipocytes. Adipocytes are specialized for the synthesis, storage and mobilization of triglycerides. The glycogen and triglyceride metabolism is highly regulated and their interplay is essential for the energy homeostasis of the body. A high glucose level in the adipocytes results in the synthesis of triglycerides as fuel store. A low intracellular glucose level leads to a release of fatty acids, which can be used as substrates for the beta-oxidation to generate energy. Glycogen levels in cells are more variable than triglyceride levels because the turnover of glycogen is higher. Triglycerides are used as long term energy donors once the glycogen stores run low.

Pancreatic beta-cells secrete insulin in response to blood glucose levels. Insulin amongst other hormones plays a key role in the regulation of the fuel metabolism. Insulin leads to the storage of glycogen and triglycerides and to the synthesis of proteins. The entry of glucose into muscles and adipose cells is stimulated by insulin. In patients who suffer from diabetes mellitus type I or LADA (latent autoimmue diabetes in adults (Pozzilli & Di Mario, 2001, Diabetes Care. 8:1460-67) beta-cells are being destroyed due to autoimmune attack. The amount of insulin produced by the remaining pancreatic islet cells is too low, resulting in elevated blood glucose levels (hyperglycemia). In diabetes type II liver and muscle cells loose their ability to respond to normal blood insulin levels (insulin resistance). High blood glucose levels (and also high blood lipid levels) in turn lead to an impairment of beta-cell function and to an increase in beta-cell apoptosis. It is interesting to note that the rate of beta-cell neogenesis does not appear to change in type II diabetics (Butler et al., 2003 supra), thus causing a reduction in total beta-cell mass over time. Eventually the application of exogenous insulin becomes necessary in type II diabetics.

Improving metabolic parameters such as blood sugar and blood lipid levels (e.g. through dietary changes, exercise, medication or combinations thereof) before beta-cell mass has fallen below a critical threshold leads to a relatively rapid restoration of beta-cell function. However, after such a treatment the pancreatic endocrine function would remain impaired due to the only slightly increased regeneration rate.

In type I diabetics, where beta-cells are being destroyed by autoimmune attack, treatments have been devised which modulate the immune system and may be able to stop or strongly reduce islet destruction (Raz et al., 2001, Lancet 358: 1749-1753; Chatenoud et al., 2003, Nat Rev Immunol. 3: 123-132; Homann et al., Immunity. 2002, 3:403-15). However, due to the relatively slow regeneration of human beta-cells such treatments can only be successful if they are combined with agents that can stimulate beta-cell regeneration.

Diabetes is a very disabling disease, because today's common anti-diabetic drugs do not control blood sugar levels well enough to completely prevent the occurrence of high and low blood sugar levels. Out of range blood sugar levels are toxic and cause long-term complications like for example renopathy, retinopathy, neuropathy and peripheral vascular disease. There is also a host of related conditions, such as obesity, hypertension, heart disease and hyperlipidemia, for which persons with diabetes are substantially at risk.

Apart from the impaired quality of life for the patients, the treatment of diabetes and its long term complications presents an enormous financial burden to our healthcare systems with rising tendency.Thus, for the treatment of, type I and type II diabetes as well as for latent autoimmune diabetes in adults (LADA) there is a strong need in the art to identify factors that induce regeneration of pancreatic insulin producing beta-cells. These factors could restore normal function of the endocrine pancreas once its function is impaired or event could prevent the development or progression of diabetes type I, diabetes type II, or LADA.

The concept of 'metabolic syndrome' (syndrome x, insulin-resistance syndrome, deadly quartet) was first described 1966 by Camus and reintroduced 1988 by Reaven (Camus JP, 1966, Rev Rhum Mal Osteoartic 33(1):10-14; Reaven et al. 1988, Diabetes, 37(12):1595-1607). Today metabolic syndrome is commonly defined as clustering of cardiovascular risk factors like hypertension, abdominal obesity, high blood levels of triglycerides and fasting glucose as well as low blood levels of HDL cholesterol. Insulin resistance greatly increases the risk of developing the metabolic syndrome (Reaven, 2002, Circulation 106(3): 286-288). The metabolic syndrome often precedes the development of type II diabetes and cardiovascular disease (McCook, 2002, JAMA 288: 2709-2716). The control of blood lipid levels and blood glucose levels is the essential for the treatment of the metabolic syndrome (see, for example, Santomauro A. T. et al., (1999) Diabetes, 48(9): 1836-1841).

The molecular factors regulating food intake and body weight balance are incompletely understood. Even if several candidate genes have been described which are supposed to influence the homeostatic system(s) that regulate body mass/weight, like leptin or the peroxisome proliferator-activated receptor-gamma co-activator, the distinct molecular mechanisms and/or molecules influencing obesity or body weight/body mass regulations are not known.

Secreted proteins are a major target for drug action and development. Accordingly, it is valuable to the field of pharmaceutical development to identify and characterize novel functions for secreted proteins. The present invention advances the state of the art by providing previously unknown functions for a human secreted protein.

The technical problem underlying the present invention was to provide for means and methods for modulating (pathological) metabolic conditions influencing body-weight regulation and/or energy homeostatic circuits. Also the technical problem was to provide methods for treating diabetes. The solution to said technical problems is achieved by providing the embodiments characterized in the claims.

Accordingly, the present invention relates to genes encoding secreted proteins with novel functions in body-weight regulation, energy homeostasis, metabolism, obesity and regeneration processes. The present invention discloses a specific gene involved in the regulation of body-weight, energy homeostasis, metabolism, and obesity, as well as related diseases such as diabetes mellitus, eating disorder, cachexia, hypertension, coronary heart disease, hypercholesterolemia (dyslipidemia), and/or gallstones. The present invention describes saposin-related genes, their homologous genes and proteins encoded thereby, in particular human saposin-related genes and proteins (also referred to as prosaposin, variant Gaucher disease and variant metachromatic leukodystrophy; PSAP; human pulmonary surfactant-associated protein B (SFTPB); human hypothetical protein FLJ40379), respectively, as being involved in the conditions mentioned above. We disclose a novel use of prosaposin to stimulate the formation or regeneration of insulin-producing beta cells and thus, a use in the treatment and prevention of diseases going along with (e.g. caused by, associated with or accompanied by) impaired beta-cell function, for example but not limited to diabetes mellitus. More particularly, the diseases are diabetes type I, diabetes type II or LADA.

So far, it has not been described that the proteins of the invention and homologous proteins are involved in the regulation of energy homeostasis and body-weight regulation or in regeneration processes and related disorders, and thus, no functions in metabolic diseases and dysfunctions and other diseases as listed above have been discussed.

In this invention we refer to the proteins encoded by Drosophila Saposin-related genes and homologous proteins, preferably human and murine homologous polypeptides or proteins or sequences encoding these proteins as saposin-related or as proteins of the invention.

The present invention discloses that saposin-related proteins are regulating the energy homeostasis and fat metabolism especially the metabolism and storage of triglycerides and glycogen, and polynucleotides, which identify and encode the proteins disclosed in this invention. The invention also relates to vectors, host cells, antibodies, and recombinant methods for producing the polypeptides and polynucleotides of the invention. The invention also relates to the use of these polynucleotides, polypeptides in the diagnosis, study, prevention, and treatment of metabolic diseases or dysfunctions, for example, but not limited to, metabolic syndrome, obesity, diabetes mellitus, eating disorder, cachexia, hypertension, coronary heart disease, hypercholesterolemia (dyslipidemia), and/or gallstones.

The present invention is also based on the finding that prosaposin stimulates the differentiation of insulin producing cells from stem cells in vitro. Thus, a therapeutically effective amount of prosaposin product may be administered to promote the regeneration of pancreatic beta-cells or to promote the formation of insulin-producing cells from stem cells or progenitor cells in vitro or in vivo. The present invention further relates to applications in the medical field that directly arise from the method of the invention. Additionally, the present invention relates to applications for the identification and characterization of compounds with therapeutic medical effects or toxicological effects that directly arise from the method of the invention.

Saposin-related homologous proteins and nucleic acid molecules coding therefore are obtainable from insect or vertebrate species, e.g. mammals or birds. Particularly preferred are nucleic acids encoding the human saposin-related homologs and the proteins encoded thereby (in particular human prosaposin [PSAP], human pulmonary surfactant-associated protein B [SFTPB], and/or human hypothetical protein FLJ40379).

The glycoprotein Prosaposin (PSAP) is a precursor for four saposins (A, B, C, and D) found in different cellular locations (Morimoto S. et al., (1989) Proc. Nat. Acad. Sci. 86: 3389-3393). Saposins are small lysosomal proteins that serve as activators of various lysosomal lipid-degrading enzymes (Munford R.S. et al., (1995) J. Lipid Res. 36: 1653-1663). Prosaposin occurs in various kinds of human secretory fluids such as cerebrospinal fluid, semen, milk, pancreatic juice, and bile. Prosaposin is postulated to mediate neurotrophic signaling events capable of inducing neural differentiation and preventing cell death.

A mutant mouse line in which the sphingolipid activator protein gene has been inactivated by homologous recombination technology showed complex clinical, pathologic, and biochemical abnormalities similar to those of human patients with total saposin deficiency (Fujita N. et al., (1996) Hum Mol Genet. 5: 711-725). The main pathology in the brain of affected Sap -/- mice was hypomyelination and storage of ceramides and gangliosides. Ceramide storage occurred also in brain, liver, and kidney, and ceramide catabolism is abnormally slow in fibroblasts. Examination of reproductive organs in prosaposin homozygous mutant males shows several abnormalities, indicating that prosaposin is also involved in the development and maintenance of male reproductive organs (Morales C.R. et al., (2000) J Androl 21: 765-775).

Pulmonary surfactant is a lipid-rich material that prevents lung collapse by lowering surface tension at the air-liquid interface in the alveoli of lung. It is composed primarily of phospholipids, cholesterol and proteins, including four surfactant associated proteins, two collagenous, carbohydrate-binding glycoproteins (PSP-A and PSP-D), and two small hydrophobic proteins (PSP-B and PSP-C). Pulmonary surfactant proteins are involved in respiration, and promote alveolar stability by lowering the surface tension at the air-liquid interface in the peripheral air spaces. PSP-B is a clinically important, developmentally regulated gene. Deficiency of PSPB B was demonstrated in congenital alveolar proteinosis (Nogee et al., (1993) N Engl J Med. 328: 406-410).

The invention particularly relates to a nucleic acid molecule encoding a polypeptide contributing to regulating the energy homeostasis and the metabolism of triglycerides and glycogen and regeneration processes, wherein said nucleic acid molecule comprises
(a) the nucleotide sequence of the Drosophila saposin-related gene or a mammalian, e.g. human saposin-related homolog (in particular human PSAP, SFTPB and/or FLJ40379), and/or a sequence complementary thereto,
(b) a nucleotide sequence which hybridizes at 50°C in a solution containing 1 x SSC and 0.1% SDS to a sequence of (a),
(c) a sequence corresponding to the sequences of (a) or (b) within the degeneration of the genetic code,
(d) a sequence which encodes a polypeptide which is at least 85%, preferably at least 90%, more preferably at least 95%, more preferably at least 98% and up to 99,6% identical to the amino acid sequences of a saposin-related protein, preferably a mammalian, e.g. human saposin-related homolog (in particular PSAP, SFTPB, and/or FLJ40379),
(e) a sequence which differs from the nucleic acid molecule of (a) to (d) by mutation and wherein said mutation causes an alteration, deletion, duplication and/or premature stop in the encoded polypeptide or
(f) a partial sequence of any of the nucleotide sequences of (a) to (e) having a length of 15-25 bases, preferably 25-35 bases, more preferably 35-50 bases and most preferably at least 50 bases.

The present invention relates to genes with novel functions in regeneration processes, body-weight regulation, energy homeostasis, metabolism, and obesity, fragments of said genes, polypeptides encoded by said genes or fragments thereof.

Further, the present invention relates to compositions for use in modulating adipogenis, for modulating, e.g. stimulating pancreatic development and/or for the regeneration of pancreatic cells or tissues, e.g. cells having exocrinous functions, such as acinar cells, centroacinar cells and/or ductal cells, and/or cells having endocrinous functions, particularly cells in Langerhans islets such as alpha-, beta-, delta-, and/or PP-cells, more particularly beta-cells.

The present invention relates to new uses and in vitro methods for stimulating and/or inducing the differentiation of progenitor cells, e.g. stem cells into insulin-producing cells or for promoting the protection, survival and/or regeneration of insulin producing cells using a prosaposin product and/or a modulator/effector thereof that influences, particularly increases the expression level or function of a prosaposin protein product.

Thus, the present invention provides uses for treating patients suffering from a disease caused by, associated with, and/or acompanied by functionally impaired and/or reduced numbers of pancreatic islet cells, particularly insulin producing beta-cells, of therapeutically effective amount of a prosaposin product or a compound that influences the prosaposin expression level or function. Functional impairment or loss of pancreatic islet cells may be due to e.g. autoimmune attack such as in diabetes type I or LADA, and/or due to cell degeneration such as in progressed diabetes type II. The products of the present invention may also be used to treat patients at risk to develop degeneration of insulin producing beta-cells to prevent the start or progress of such process.

The prosaposin product may be administered e.g. as a pharmaceutical composition, via implantation of prosaposin product expressing cells and/or via gene therapy.

Further, the invention relates to cell preparations comprising prosaposin-treated insulin producing cells or prosaposin expressing cells.

Numerous additional aspects and advantages of the invention will become apparent to those skilled in the art upon consideration of the following description of the Figures and detailed description of the invention which describes presently preferred embodiments thereof.

The ability to manipulate and screen the genomes of model organisms such as the fly Drosophila melanogaster provides a powerful tool to analyze biological and biochemical processes that have direct relevance to more complex vertebrate organisms due to significant evolutionary conservation of genes, cellular processes, and pathways (see, for example, Adams M. D. et al., (2000) Science 287: 2185-2195). Identification of novel gene functions in model organisms can directly contribute to the elucidation of correlative pathways in mammals (humans) and of methods of modulating them. A correlation between a pathology model (such as changes in triglyceride levels as indication for metabolic syndrome including obesity) and the modified expression of a fly gene can identify the association of the human ortholog with the particular human disease.

A forward genetic screen is performed in fly displaying a mutant phenotype due to misexpression of a known gene (see, Johnston Nat Rev Genet 3: 176-188 (2002); Rorth P., (1996) Proc Natl Acad Sci U S A 93: 12418-12422). In this invention, we have used a genetic screen to identify mutations of Saposin-related homologous genes that cause changes in the body weight, which are reflected by a significant change of triglyceride levels. Additionally glycogen levels are analysed.

One resource for screening was a Drosophila melanogaster stock collection of EP-lines. The P-vector of this collection has Gal4-UAS-binding sites fused to a basal promoter that can transcribe adjacent genomic Drosophila sequences upon binding of Gal4 to UAS-sites (Brand & Perrimon (1993) Development 118: 401-415; Rorth P., supra). This enables the EP-line collection for overexpression of endogenous flanking gene sequences. In addition, without activation of the UAS-sites, integration of the EP-element into the gene is likely to cause a reduction of gene activity, and allows determining its function by evaluating the loss-of-function phenotype.

To isolate genes with a function in energy homeostasis, several thousand EP-lines were tested for their triglyceride/glycogen content after a prolonged feeding period (see Examples for more detail). Lines with significantly changed triglyceride/glycogen content were selected as positive candidates for further analysis. The change of triglyceride/glycogen content due to the loss of a gene function suggests gene activities in energy homeostasis in a dose dependent manner that control the amount of energy stored as triglycerides or glycogen.

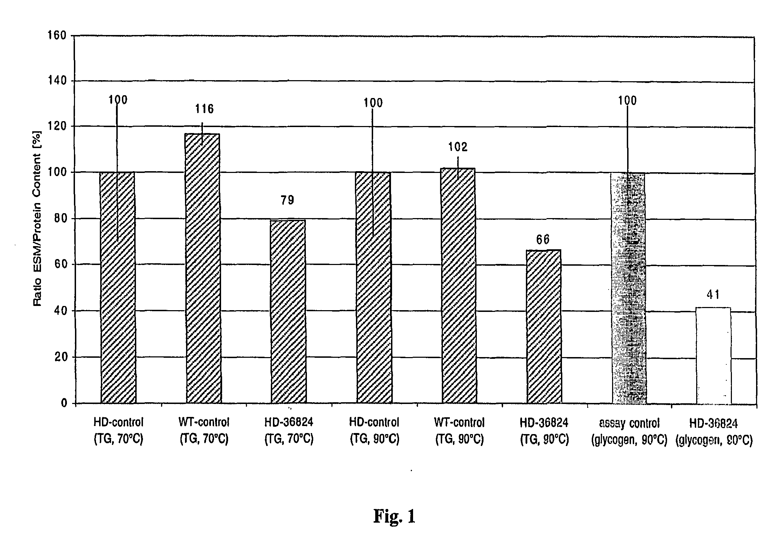

In this invention, the content of triglycerides and glycogen of a pool of flies with the same genotype was analyzed after feeding for six days using a triglyceride and a glycogen assay. Male flies homozygous for the integration of vectors for Drosophila lines HD-EP(3)36824 were analyzed in assays measuring the triglyceride/glycogen contents of these flies (illustrated in more detail in the Examples section). The results of the triglyceride/glycogen content analysis are shown in Figure 1.

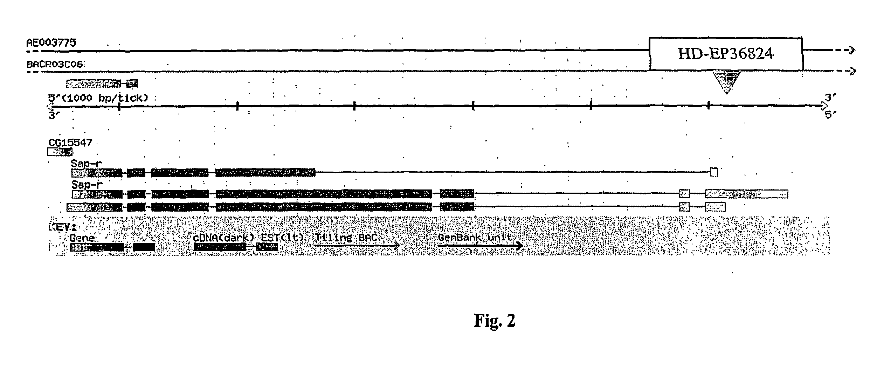

Genomic DNA sequences were isolated that are localized directly adjacent to the EP vector (herein HD-EP(3)36824) integration. Using those isolated genomic sequences public databases like Berkeley Drosophila Genome Project (GadFly; see also FlyBase (1999) Nucleic Acids Research 27:85-88) were screened thereby identifying the integration site of the vectors, and the corresponding gene, described in more detail in the Examples section. The molecular organization of the gene is shown in Figure 2.

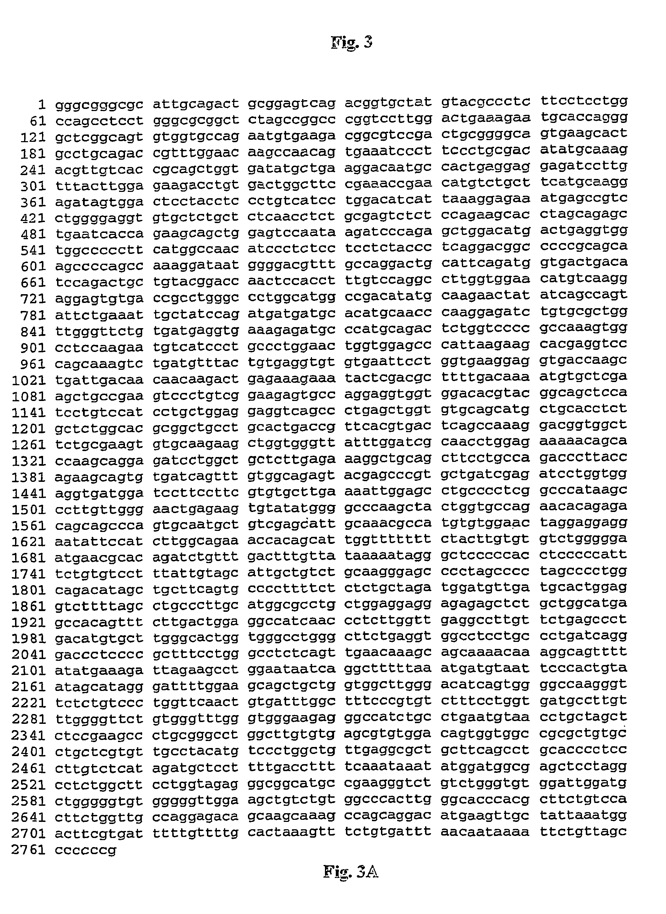

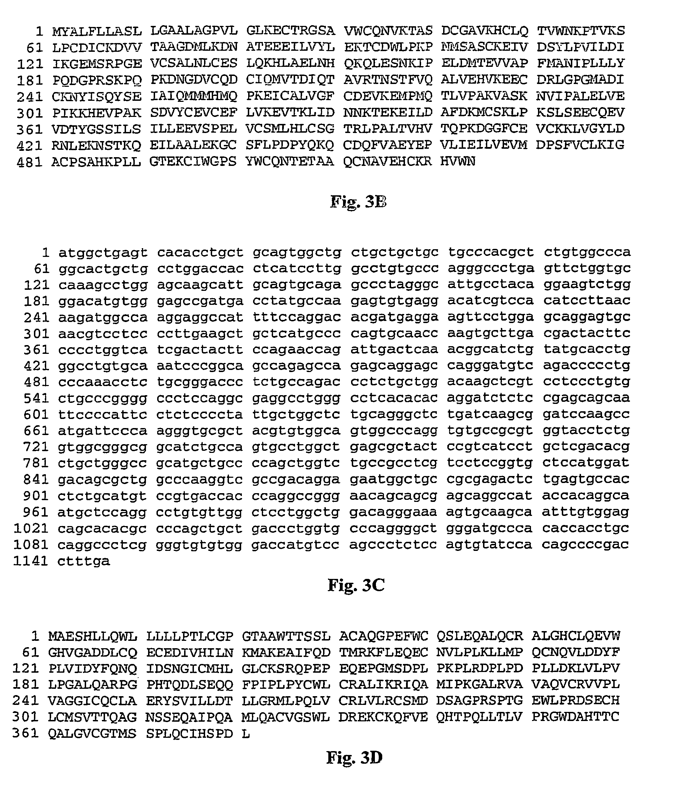

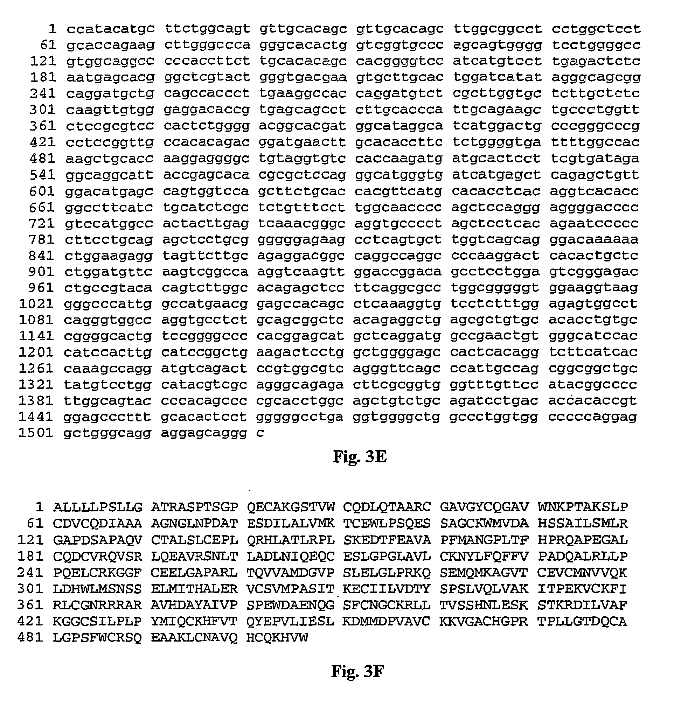

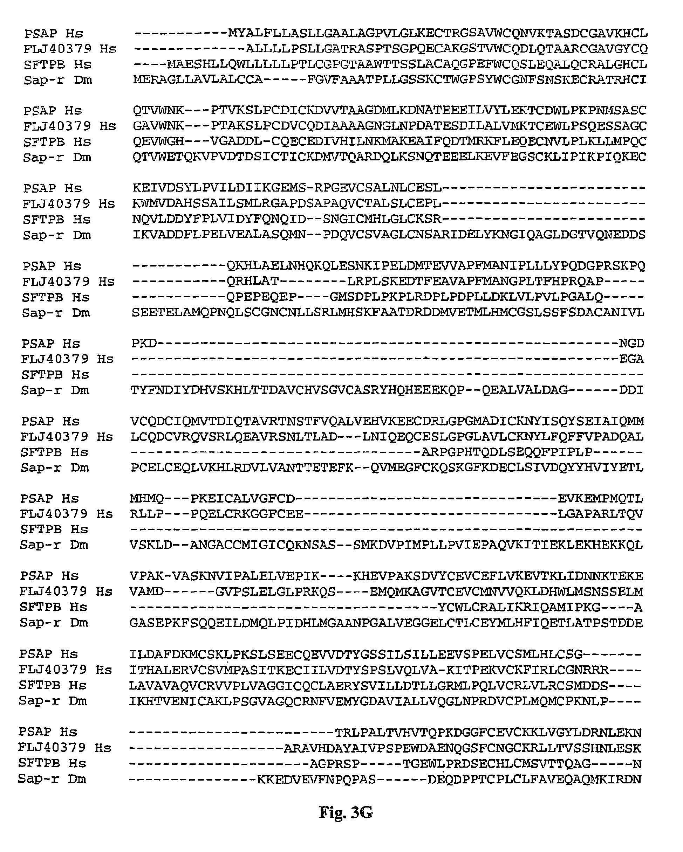

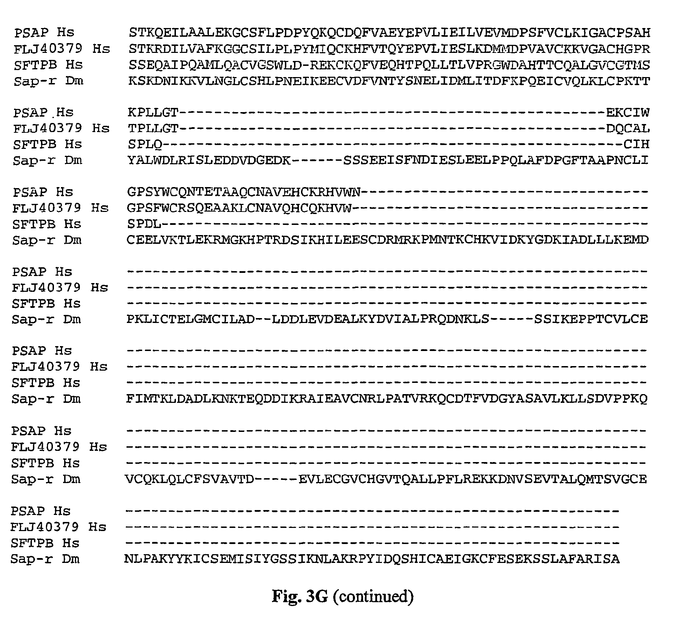

The Drosophila genes and proteins encoded thereby with functions in the regulation of triglyceride metabolism were further analysed in publicly available sequence databases (see Eamples for more detail) and mammalian homologs were identified (see Figure 3).

In addition, we identified prosaposin (PSAP) as secreted factor expressed in developing mouse pancreas, as described in more detail in the Examples section.

The function of the mammalian homologs in energy homeostasis was further validated in this invention by analyzing the expression of the transcripts in different tissues and by analyzing the role in adipocyte differentiation.

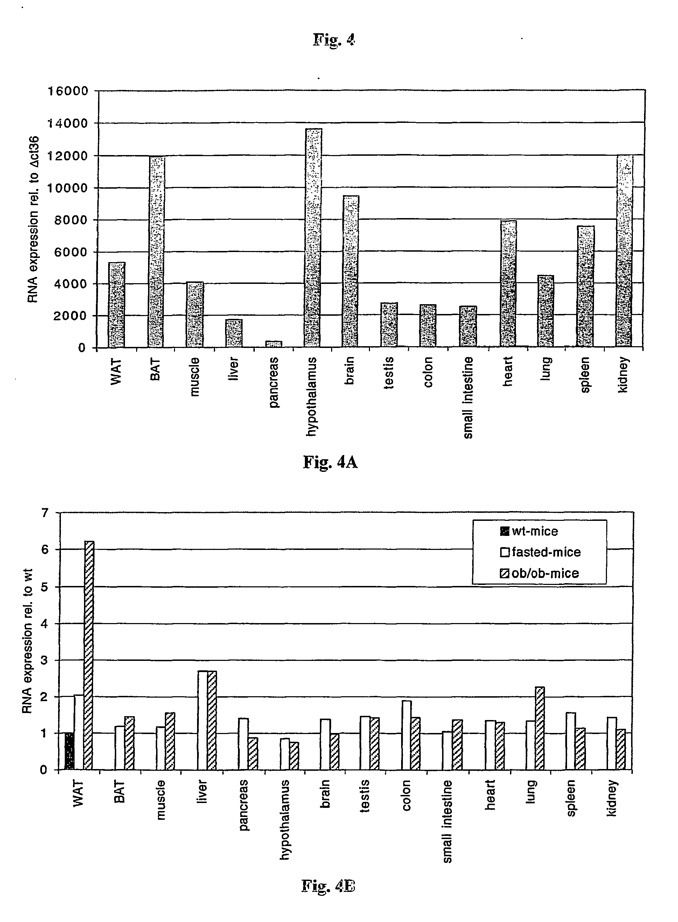

Expression profiling studies (see Examples for more detail) confirm the particular relevance of the protein of the invention as regulators of energy metabolism in mammals. Transcripts of prosaposin are found in several tissues of mammals with high expression levels in brain tissues (hypothalamus), in kidney, heart and spleen. In addition, prosaposin shows high expression in brown adipose tissue (BAT) and white adipose tissue (WAT) (see FIGURE 4A). Brown adipose tissue is a well-characterized tissue which is well developed in newborn mammals, including humans. One important task of BAT is to generate heat and maintain body temperature homeostasis in newborn. Thus, an expression of the protein of the invention in adipose tissues is confirming a role in the regulation of energy homeostasis and thermogenesis.

Further, we show that mammalian prosaposin is regulated by fasting and by genetically induced obesity. In this invention, we used mouse models of insulin resistance and/or diabetes, such as mice carrying gene knockouts in the leptin pathway (for example, ob (leptin) or db (leptin receptor) mice) to study the expression of prosaposin. Such mice develop typical symptoms of diabetes, show hepatic lipid accumulation and frequently have increased plasma lipid levels (see Bruning et al, 1998, Mol. Cell. 2: 559-569). We found, for example, that the expression of prosaposin is strongly upregulated in liver of fasted and ob/ob mice (see Figure 4B). In addition, a marked upregulation can be observed in the metabolically active tissue (for example, white adipose tissue (WAT)) of genetically obese (ob/ob) (see FIGURE 4B).

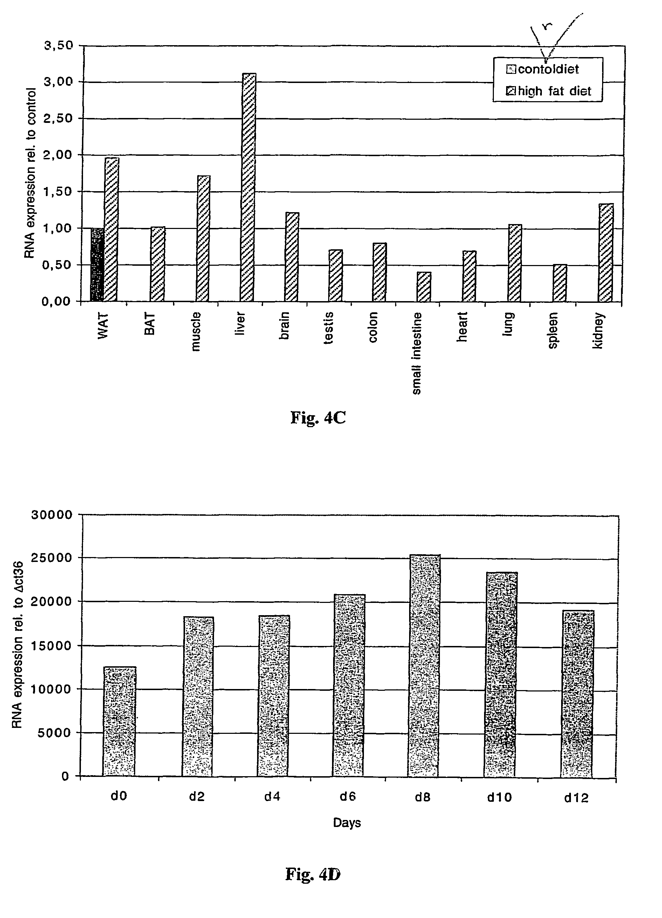

Susceptible wild type mice (for example C57BI/6) show symptoms of diabetes, lipid accumulation, and high plasma lipid levels, if fed a high fat diet. In such mice, the most prominent response with regard to metabolically active tissues was observed. In those mice, the expression of prosaposin is significantly enhanced in white adipose tissue (see Figure 4C) and in liver and muscle tissues, supporting a hypothesis that the protein of the invention is a modulator of adipogenesis.

In addition, we show in this invention that the RNA of the protein of the invention is up-regulated during adipocyte differentiation in vitro (see EXAMPLES for more detail), suggesting a role as modulator of adipocyte lipid accumulation. With regard to changes in expression intensity during the differentiation of preadipocytes to adipocytes, an enhancement in relative signal intensity can be observed for the protein of the invention during the in vitro differentiation program of 3T3-L1 (see Figure 4D). Thus, we conclude that prosaposin or variants or processing products thereof have a function in the metabolism of mature adipocytes.

Microarrays are analytical tools routinely used in bioanalysis. A microarray has molecules distributed over, and stably associated with, the surface of a solid support. The term "microarray" refers to an arrangement of a plurality of polynucleotides, polypeptides, antibodies, or other chemical compounds on a substrate. Microarrays of polypeptides, polynucleotides, and/or antibodies have been developed and find use in a variety of applications, such as monitoring gene expression, drug discovery, gene sequencing, gene mapping, bacterial identification, and combinatorial chemistry. One area in particular in which microarrays find use is in gene expression analysis (see Example 4). Array technology can be used to explore the expression of a single polymorphic gene or the expression profile of a large number of related or unrelated genes. When the expression of a single gene is examined, arrays are employed to detect the expression of a specific gene or its variants. When an expression profile is examined, arrays provide a platform for identifying genes that are tissue specific, are affected by a substance being tested in a toxicology assay, are part of a signaling cascade, carry out housekeeping functions, or are specifically related to a particular genetic predisposition, condition, disease, or disorder.

Microarrays may be prepared, used, and analyzed using methods known in the art (see for example, Brennan, T.M. et al. (1995) U.S. Patent No. 5,474,796, Schena, M. et al. (1996) Proc. Natl. Acad. Sci. USA 93: 10614-10619; Baldeschweiler et al. (1995) PCT application WO95/251116; Shalon, D. et al. (1995) PCT application WO95/35505; Heller, R.A. et al. (1997) Proc. Natl. Acad. Sci. USA 94:21502155; Heller, M.J. et al. (1997) U.S. Patent No. 5,605,662). Various types of microarrays are well known and thoroughly described in Schena, M., ed. (1999; DNA Microarrays: A Practical Approach, Oxford University Press, London).

Oligonucleotides or lounger fragments derived from any of the polynucleotides described herein may be used as elements on a microarray. The microarray can be used in transcript imaging techniques which monitor the relative expression levels of large numbers of genes simultaneously as described below. The microarray may also be used to identify genetic variants, mutations, and polymorphisms. This information may be used to determine gene function, to understand the genetic basis of a disorder, to diagnose a disorder, to monitor progression/regression of disease as a function of gene expression, and to develop and monitor the activities of therapeutic agents in the treatment of disease. In particular, this information may be used to develop a pharmacogenomic profile of a patient in order to select the most appropriate and effective treatment regimen for that patient. For example, therapeutic agents which are highly effective and display the fewest side effects may be selected for a patient based on his/her pharmacogenomic profile.

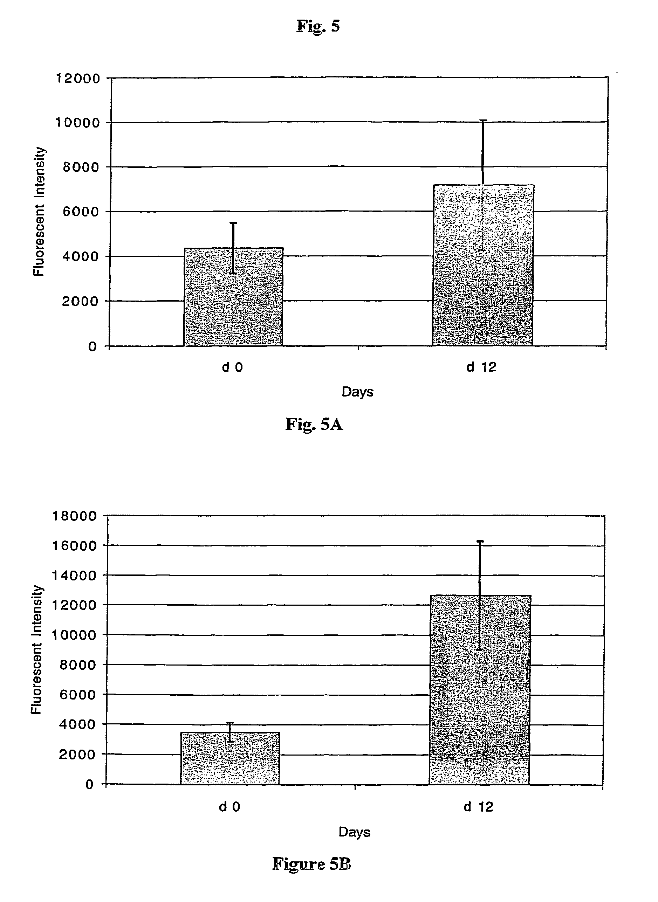

As determined by microarray analysis, prosaposin shows differential expression in human primary adipocytes (see Figure 5A) and a human adipocyte cell line (see Figure 5B). The expression of prosaposin is strongly upregulated during the human adipocyte differentiation (see FIGURE 5). Thus, the prosaposin protein in preadipocyctes is signifficantly enhancing adipose differentiation at a very early stage. Therefore, prosaposin plays an essential role in adipogenesis. In addition, the results strongly suggest a role of prosaposin in the regulation in human metabolism, for example, as modulator (for example, enhancer) of adipogenesis. Thus, prosaposin is a strong candidate for a pharmaceutical composition for the treatment of conditions related to human metabolism, such as obesity, diabetes, and/or metabolic syndrome.

This invention further shows that prosaposin induces the differentiation of insulin-producing cells and is thus a target for the treatment of diabetes. In connection with the present invention, the term "progenitor cells" relates to undifferentiated cells capable of being differentiated into insulin producing cells. The term particularly includes stem cells, i.e. undifferentiated or immature embryonic, adult, or somatic cells that can give rise to various specialized cell types. The term "stem cells" can include embryonic stem cells (ES) and primordial germ cells (EG) cells of mammalian, e.g. human or animal origin. Isolation and culture of such cells is well known to those skilled in the art (see, for example, Thomson et al., (1998) Science 282: 1145-1147; Shamblott et al., (1998) Proc. Natl. Acad. Sci. USA 95: 13726-13731; US 6,090,622; US 5,914,268; WO 00/27995; Notarianni et al., (1990) J. Reprod. Fert. 41: 51-56; Vassilieva et al., (2000) Exp. Cell. Res. 258: 361-373). Adult or somatic stem cells have been identified in numerous different tissues such as intestine, muscle, bone marrow, liver, and brain. WO 03/023018 describes a novel method for isolating, culturing, and differentiating intestinal stem cells for therapeutic use. In the pancreas, several indications suggest that stem cells are also present within the adult tissue (Gu and Sarvetnick, (1993) Development 118: 33-46; Bouwens, (1998) Microsc Res Tech 43: 332-336; Bonner-Weir, (2000) J. Mol. Endocr. 24: 297-302).

Embryonic stem cells can be isolated from the inner cell mass of pre-implantation embryos (ES cells) or from the primordial germ cells found in the genital ridges of post-implanted embryos (EG cells). When grown in special culture conditions such as spinner culture or hanging drops, both ES and EG cells aggregate to form embryoid bodies (EB). EBs are composed of various cell types similar to those present during embryogenesis. When cultured in appropriate media, EB can be used to generate in vitro differentiated phenotypes, such as extraembryonic endoderm, hematopoietic cells, neurons, cardiomyocytes, skeletal muscle cells, and vascular cells. We have previously described a method that allows EB to efficiently differentiate into insulin-producing cells (as described in patent application PCT/EP02/04362, published as WO 02/086107 and by Blyszczuk et al., (2003) Proc Natl Acad Sci USA 100: 998-1003).

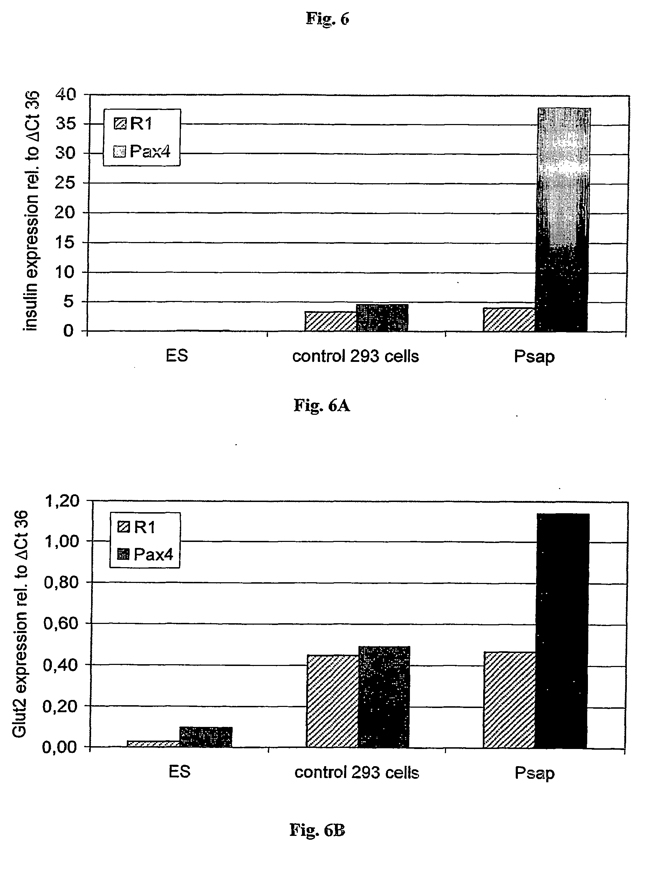

The results shown in Figure 6 clearly demonstrate an induction of the differentiation of insulin-producing and glucose-responsive cells by prosaposin. Thus, prosaposin can induce the differentiation of beta-cells and is therefore a target for therapeutic uses in the treatment of diabetes, for example, when regeneration of cells is required.

Before the present invention is described, it is understood that all technical and scientific terms used herein have the same meanings as commonly understood by one of ordinary skill in the art to which this invention belongs.

In the present invention the term "beta-cell regeneration" refers to an at least partial restoration of normal beta-cell function by increasing the number of functional insulin secreting beta-cells and/or by restoring normal function in functionally impaired beta-cells.

The invention also encompasses polynucleotides that encode the proteins of the invention and homologous proteins. Accordingly, any nucleic acid sequence, which encodes the amino acid sequences of the proteins of the invention and homologous proteins, can be used to generate recombinant molecules that express the proteins of the invention and homologous proteins. In a particular embodiment, the invention encompasses a nucleic acid encoding Drosophila saposin-related, or their mammalian, e.g. human homologs; referred to herein as the proteins of the invention. It will be appreciated by those skilled in the art that as a result of the degeneracy of the genetic code, a multitude of nucleotide sequences encoding the proteins, some bearing minimal homology to the nucleotide sequences of any known and naturally occurring gene, may be produced. The invention contemplates each and every possible variation of nucleotide sequence that can be made by selecting combinations based on possible codon choices.

Also encompassed by the invention are polynucleotide sequences that are capable of hybridizing to the claimed nucleotide sequences, and in particular, those of the polynucleotide encoding the proteins of the invention, under various conditions of stringency. Hybridization conditions are based on the melting temperature (Tm) of the nucleic acid binding complex or probe, as taught in Wahl & Berger (1987: Methods Enzymol. 152: 399-407) and Kimmel (1987; Methods Enzymol. 152: 507-511), and may be used at a defined stringency. Preferably, hybridization under stringent conditions means that after washing for 1 h with 1 x SSC and 0.1% SDS at 50°C, preferably at 55°C, more preferably at 62°C and most preferably at 65°C, particularly for 1 h in 0.2 x SSC and 0.1% SDS at 50°C, preferably at 55°C, more preferably at 62°C and most preferably at 65°C, a positive hybridization signal is observed. Altered nucleic acid sequences encoding the proteins which are encompassed by the invention include deletions, insertions or substitutions of different nucleotides resulting in a polynucleotide that encodes the same or a functionally equivalent protein.

The encoded proteins may also contain deletions, insertions or substitutions of amino acid residues, which produce a silent change and result in functionally equivalent proteins. Deliberate amino acid substitutions may be made on the basis of similarity in polarity, charge, solubility, hydrophobicity, hydrophilicity, and/or the amphipathic nature of the residues as long as the biological activity of the protein is retained. Furthermore, the invention relates to peptide fragments of the proteins or derivatives thereof such as cyclic peptides, retro-inverso peptides or peptide mimetics having a length of at least 4, preferably at least 6 and up to 50 amino acids.

Also included within the scope of the present invention are alleles of the genes encoding the proteins of the invention and homologous proteins. As used herein, an 'allele' or 'allelic sequence' is an alternative form of the gene, which may result from at least one mutation in the nucleic acid sequence. Alleles may result in altered mRNAs or polypeptides whose structures or function may or may not be altered. Any given gene may have none, one or many allelic forms. Common mutational changes, which give rise to alleles, are generally ascribed to natural deletions, additions or substitutions of nucleotides. Each of these types of changes may occur alone or in combination with the others, one or more times in a given sequence.

The nucleic acid sequences encoding the proteins of the invention and homologous proteins may be extended utilizing a partial nucleotide sequence and employing various methods known in the art to detect upstream sequences such as promoters and regulatory elements.

In order to express a biologically active protein, the nucleotide sequences encoding the proteins or functional equivalents, may be inserted into appropriate expression vectors, i.e., a vector which contains the necessary elements for the transcription and translation of the inserted coding sequence. Methods, which are well known to those skilled in the art, may be used to construct expression vectors containing sequences encoding the proteins and the appropriate transcriptional and translational control elements. Regulatory elements include for example a promoter, an initiation codon, a stop codon, a mRNA stability regulatory element, and a polyadenylation signal. Expression of a polynucleotide can be assured by (i) constitutive promoters such as the Cytomegalovirus (CMV) promoter/enhancer region, (ii) tissue specific promoters such as the insulin promoter (see, Soria et al., 2000, Diabetes 49: 157-162), SOX2 gene promotor (see Li et al., (1998) Curr. Biol. 8: 971-974), Msi-1 promotor (see Sakakibara et al., (1997) J. Neuroscience 17: 8300-8312), alpha-cardia myosin heavy chain promotor or human atrial natriuretic factor promotor (Klug et al., (1996) J. Clin. Invest 98: 216-224; Wu et al., (1989) J. Biol. Chem. 264: 6472-6479) or (iii) inducible promoters such as the tetracycline inducible system. Expression vectors can also contain a selection agent or marker gene that confers antibiotic resistance such as the neomycin, hygromycin or puromycin resistance genes. These methods include in vitro recombinant DNA techniques, synthetic techniques, and in vivo genetic recombination. Such techniques are described in Sambrook, J. et al. (1989) Molecular Cloning, A Laboratory Manual, Cold Spring Harbor Press, Plainview, N.Y. and Ausubel, F.M. et al. (1989) Current Protocols in Molecular Biology, John Wiley & Sons, New York, N.Y.

In a further embodiment of the invention, natural, modified or recombinant nucleic acid sequences encoding the proteins of the invention and homologous proteins may be ligated to a heterologous sequence to encode a fusion protein.

A variety of expression vector/host systems may be utilized to contain and express sequences encoding the proteins or fusion proteins. These include, but are not limited to, micro-organisms such as bacteria transformed with recombinant bacteriophage, plasmid or cosmid DNA expression vectors; yeast transformed with yeast expression vectors; insect cell systems infected with virus expression vectors (e.g., baculovirus, adenovirus, adeno-associated virus, lentiverus, retrovirus); plant cell systems transformed with virus expression vectors (e.g., cauliflower mosaic virus, CaMV; tobacco mosaic virus, TMV) or with bacterial expression vectors (e.g., Ti or PBR322 plasmids); or animal cell systems.

The presence of polynucleotide sequences of the invention in a sample can be detected by DNA-DNA or DNA-RNA hybridization and/or amplification using probes or portions or fragments of said polynucleotides. Nucleic acid amplification based assays involve the use of oligonucleotides or oligomers based on the sequences specific for the gene to detect transformants containing DNA or RNA encoding the corresponding protein. As used herein 'oligonucleotides' or 'oligomers' refer to a nucleic acid sequence of at least about 10 nucleotides and as many as about 60 nucleotides, preferably about 15 to 30 nucleotides, and more preferably about 20-25 nucleotides, which can be used as a probe or amplimer.

A wide variety of labels and conjugation techniques are known by those skilled in the art and may be used in various nucleic acid and amino acid assays. Means for producing labeled hybridization or PCR probes for detecting polynucleotide sequences include oligo-labeling, nick translation, end-labeling of RNA probes, PCR amplification using a labeled nucleotide, or enzymatic synthesis. These procedures may be conducted using a variety of commercially available kits (Pharmacia & Upjohn, (Kalamazoo, Mich.); Promega (Madison Wis.); and U.S. Biochemical Corp., (Cleveland, Ohio).

The presence of proteins of the invention in a sample can be determined by immunological methods or activity measurement. A variety of protocols for detecting and measuring the expression of proteins, using either polyclonal or monoclonal antibodies specific for the protein or reagents for determining protein activity are known in the art. Examples include enzyme-linked immunosorbent assay (ELISA), radioimmunoassay (RIA), and fluorescence activated cell sorting (FACS). A two-site, monoclonal-based immunoassay utilizing monoclonal antibodies reactive to two non-interfering epitopes on the protein is preferred, but a competitive binding assay may be employed. These and other assays are described, among other places, in Hampton, R. et al. (1990; Serological Methods, a Laboratory Manual, APS Press, St Paul, Minn.) and Maddox, D. E. et al. (1983; J. Exp. Med. 158: 1211-1216).

Suitable reporter molecules or labels, which may be used, include radionuclides, enzymes, fluorescent, chemiluminescent or chromogenic agents as well as substrates, co-factors, inhibitors, magnetic particles, and the like.

Host cells transformed with nucleotide sequences encoding a protein of the invention may be cultured under conditions suitable for the expression and recovery of said protein from cell culture. The protein produced by a recombinant cell may be secreted or contained intracellularly depending on the sequence or/and the vector used. As will be understood by those of skill in the art, expression vectors containing polynucleotides, which encode the protein may be designed to contain signal sequences, which direct secretion of the protein through a prokaryotic or eukaryotic cell membrane. Other recombinant constructions may be used to join sequences encoding the protein to nucleotide sequence encoding a polypeptide domain, which will facilitate purification of soluble proteins. Such purification facilitating domains include, but are not limited to, metal chelating peptides such as histidine-tryptophan modules that allow purification on immobilized metals, protein A domains that allow purification on immobilized immunoglobulin, and the domain utilized in the FLAG extension/affinity purification system (Immunex Corp., Seattle, Wash.) The inclusion of cleavable linker sequences such as those specific for Factor XA or Enterokinase (Invitrogen, San Diego, Calif.) between the purification domain and the desired protein may be used to facilitate purification.

### Diagnostics and Therapeutics

The data disclosed in this invention show that the nucleic acids and proteins of the invention and effector/modulator molecules thereof are useful in diagnostic and therapeutic applications implicated, for example, but not limited to, metabolic syndrome including obesity, diabetes mellitus, eating disorder, cachexia, hypertension, coronary heart disease, hypercholesterolemia (dyslipidemia), and / or gallstones. Hence, diagnostic and therapeutic uses for the proteins of the invention nucleic acids and proteins of the invention are, for example but not limited to, the following: (i) tissue regeneration in vitro and in vivo (regeneration for all these tissues and cell types composing these tissues and cell types derived from these tissues), (ii) small molecule drug target, (iii) antibody target (therapeutic, diagnostic, drug targeting/cytotoxic antibody), (iv) diagnostic and/or prognostic marker, (v) protein therapy, (vi) gene therapy (gene delivery/gene ablation), and (vii) research tools.

According to this invention the prosaposin product may be administered
i) as a pharmaceutical composition e.g. enterally, parenterally or topically, preferably directly to the pancreas,
ii) via implantation of prosaposin protein product expressing cells, and/or
iii) via gene therapy
as described in more detail below.

Further, the prosaposin expression level in a patient might be influenced by a prosaposin modulator/effector administered
i) as a pharmaceutical composition e.g. enterally, parenterally or topically, preferably directly to the pancreas,
ii) via cell based therapy, and/or
iii) via gene therapy
as described in more detail below.

The prosaposin product may be administered in the above described manner alone or in combination with another pharmaceutical composition useful to treat beta-cell degeneration, for example hormones, growth factors or immune modulating agents.

A prosaposin product may be administered in patients suffering from a disease going along with impaired beta-cell function, for example but not limited to diabetes type I, LADA, or progressed diabetes type II. It is further contemplated that a prosaposin product or the modulator/effector thereof may be administered preventively to patients at risk to develop beta-cell degeneration, like for example but not limited to patients suffering from diabetes type II or LADA in early stages. A variety of pharmaceutical formulations and different delivery techniques are described in further detail below.

The present invention also relates to methods for differentiating progenitor cells into insulin-producing cells in vitro comprising
(a) activating one or more pancreatic genes in a progenitor, e.g. stem cell (optional step, particularly if embryonic stem cells are used)
(b) aggregating said cells to form embryoid bodies (optional step, particularly if embryonic stem cells are used)
(c) cultivating embryoid bodies or cultivating adult stem cells (e.g., duct cells) in specific differentiation media containing a prosaposin protein product and/or a modulator/effector thereof under conditions wherein beta-cell differentiation is significantly enhanced, and
(d) identifying and selecting insulin-producing cells.

Activation of pancreatic genes may comprise transfection of a cell with pancreatic gene operatively linked to an expression control sequence, e.g. on a suitable transfection vector, as described in WO 03/023018. Examples of preferred pancreatic genes are Pdx1, Pax4, Pax6, neurogenin 3 (ngn3), Nkx 6.1, Nkx 6.2, Nlkx 2.2, HB 9, BETA2/Meuro D, Isl 1, HNF1-alpha, HNF1-beta and HNF3 of human or animal origin. Each gene can be used individually or in combination with at least one other gene. Pax4 is especially preferred.

Prosaposin products, e.g. prosaposin protein or nucleic acid products, are preferably produced via recombinant techniques because such methods are capable of achieving high amounts of protein at a great purity, but are not limited to products expressed in bacterial, plant, mammalian, or insect cell systems.

Further, the data show that the prosaposin nucleic acids and proteins are useful for the modulation, e.g. stimulation, of pancreatic development and/or for the regeneration of pancreatic cells or tissues, e.g. cells having exocrinous functions such as acinar cells, centroacinar cells and/or ductal cells, and/or cells having endocrinous functions, particularly cells in Langerhans islets such as alpha-, beta-, delta- and/or PP-cells, more particularly beta-cells.

The nucleic acids and proteins of the invention are useful in diagnostic and therapeutic applications implicated in various applications as described below. For example, but not limited to, cDNAs encoding the proteins of the invention and particularly their human homologues may be useful in gene therapy, and the proteins of the invention and particularly their human homologues may be useful when administered to a subject in need thereof. By way of non-limiting example, the compositions of the present invention will have efficacy for treatment of patients suffering from, for example, but not limited to, in metabolic disorders as described above.

Prosaposin product cell therapy, i.e. pancreatic implantation of cells producing prosaposin protein product, is also contemplated. This embodiment would involve implanting cells capable of synthesizing and secreting a biologically active form of prosaposin protein product into patients. Such prosaposin protein product-producing cells may be cells that are natural producers of prosaposin protein product or may be cells that are modified to express the protein. Such modified cells include recombinant cells whose ability to produce a prosaposin protein product has been augmented by transformation with a gene encoding the desired prosaposin protein product in a vector suitable for promoting its expression and secretion. In order to minimize a potential immunological reaction in patients being administered prosaposin protein product of a foreign species, it is preferred that the cells producing prosaposin protein product be of human origin and produce human prosaposin protein product. Likewise, it is preferred that the recombinant cells producing prosaposin protein product be transformed with an expression vector containing a gene encoding a human prosaposin protein product. Implanted cells may be encapsulated to avoid infiltration of surrounding tissue. Human or nonhuman animal cells may be implanted in patients in biocompatible, semipermeable polymeric enclosures or membranes that allow release of prosaposin protein product, but that prevent destruction of the cells by the patient's immune system or by other detrimental factors from the surrounding tissue.

Alternatively, prosaposin protein product secreting cells may be introduced into a patient in need intraportally via a percutaneous transhepatic approach using local anaesthesia. Between 3000 and 100 000 equivalent differentiated insulin-producing cells per kilogram body weight are preferably administered. Such surgical techniques are well known in the art and can be applied without any undue experimentation, see Pyzdrowski et al, 1992, New England J. Medicine 327:220-226; Hering et al., Transplantation Proc. 26:570-571, 1993; Shapiro et al., New England J. Medicine 343:230-238, 2000.

In a further preferred embodiment, prosaposin protein product can be delivered directly to progenitor, e.g. stem cells in order to stimulate the differentiation of insulin producing cells. For example, protein delivery can be achieved by polycationic liposomes (Sells et al. (1995) Biotechniques 19:72-76), Tat-mediated protein transduction (Fawell et al. (1993) Proc. Natl. Acad. Sci. USA 91:664-668) and by fusing a protein to the cell permeable motif derived from the PreS2-domain of the hepatitis-B virus (Oess and Hildt (2000) Gene Ther. 7:750-758). Preparation, production and purification of such proteins from bacteria, yeast or eukaryotic cells are well known by persons skilled in the art. In this embodiment of the invention, prosaposin may be added preferably at concentrations between 1 ng/ml and 500 ng/ml, more preferably between 10 and 100 ng/ml, e.g. at about 50 ng/ml.

Further, the invention relates to a cell preparation comprising differentiated progenitor cells, e.g. stem cells exhibiting insulin production, particularly an insulin-producing cell line obtainable by the method described above. The insulin-producing cells may exhibit a stable or a transient expression of at least one pancreatic gene involved in beta-cell differentiation. The cells are preferably human cells that are derived from human stem cells. For therapeutic applications the production of autologous human cells from adult stem cells of a patient is especially preferred. However, the insulin producing cells may also be derived from non-autologous cells. If necessary, undesired immune reactions may be avoided by encapsulation, immunosuppression and/or modulation or due to non-immunogenic properties of the cells.

The insulin producing cells of the invention preferably exhibit characteristics that closely resemble naturally occurring beta-cells. Further, the cells of the invention preferably are capable of a quick response to glucose. After addition of 27.7 mM glucose, the insulin production is enhanced by a factor of at least 2, preferably by a factor of at least 3. Further, the cells of the invention are capable of normalizing blood glucose levels after transplantation into mice.

The invention further encompasses functional pancreatic cells obtainable or obtained by the method according to the invention. The cells are preferably of mammalian, e.g. human origin. Preferably, said cells are pancreatic beta-cells, e.g. mature pancreatic beta-cells or stem cells differentiated into pancreatic beta-cells. Such pancreatic beta cells preferably secrete insulin in response to glucose. Moreover, the present invention provides functional pancreatic cell that express glucagon in response to glucose. A preparation comprising the cells of the invention may additionally contain cells with properties of other endocrine cell types such as alpha-cells, delta-cells and/or PP-cells. These cells are preferably human cells.
The cell preparation of the invention is preferably a pharmaceutical composition comprising the cells together with pharmacologically acceptable carriers, diluents and/or adjuvants. The pharmaceutical composition is preferably used for the treatment or prevention of pancreatic diseases, e.g. diabetes.

According to the present invention, the functional insulin producing cells treated with prosaposin may be transplanted preferably intrahepatic, directly into the pancreas of an individual in need, or by other methods. Alternatively, such cells may be enclosed into implantable capsules that can be introduced into the body of an individual, at any location, more preferably in the vicinity of the pancreas, or the bladder, or the liver, or under the skin. Methods of introducing cells into individuals are well known to those of skill in the art and include, but are not limited to, injection, intravenous or parenteral administration. Single, multiple, continuous or intermittent administration can be effected. The cells can be introduced into any of several different sites, including but not limited to the pancreas, the abdominal cavity, the kidney, the liver, the celiac artery, the portal vein or the spleen. The cells may also be deposited in the pancreas of the individual.

The methodology for the membrane encapsulation of living cells is familiar to those of ordinary skill in the art, and the preparation of the encapsulated cells and their implantation in patients may be accomplished without undue experimentation. See, e.g., U.S. Patent Numbers 4,892,538, 5,011,472, and 5,106.627. A system for encapsulating living cells is described in PCT Application WO 91/10425 of Aebischer et al., See also, PCP Application WO 91/10470 of Aebischer et al., Winn et al., Exper. Neurol., 1 13:322-329, 1991, Aebischer et al., Exper. Neurol., 11 1:269-275, 1991; Tresco et al., ASAIO, 38:17-23, 1992. Techniques for formulating a variety of other sustained- or controlled-delivery means, such as liposome carriers, bio-erodible particles or beads and depot injections, are also known to those skilled in the art.

In another embodiment gene therapy ex vivo is envisioned, i.e. the patient's own cells may be transformed ex vivo to produce a prosaposin protein product or a protein stimulating prosaposin expression and would be directly reimplanted. For example, cells retrieved from the patient may be cultured and transformed with an appropriate vector. After an optional propagation/expansion phase, the cells can be transplanted back into the same patient's body, particularly the pancreas, where they would produce and release the desired prosaposin protein product. Delivery by transfection and by liposome injections may be achieved using methods, which are well known in the art. Any of the therapeutic methods described above may be applied to any suitable subject including, for example, mammals such as dogs, cats, cows, horses, rabbits, monkeys, and most preferably, humans.

Prosaposin product gene therapy in vivo is also envisioned, by introducing the gene coding for a prosaposin protein product into targeted pancreas cells via local injection of a nucleic acid construct or other appropriate delivery methods (Hefti, J. Neurobiol., 25:1418-1435, 1994). For example, a nucleic acid sequence encoding a prosaposin protein product may be contained in an adeno-associated virus vector or adenovirus vector for delivery to the pancreas cells. Alternative viral vectors include, but are not limited to, retrovirus, herpes simplex virus and papilloma virus vectors. Physical transfer, either in vivo or ex vivo as appropriate, may also be achieved by liposome-mediated transfer, direct injection (naked DNA), receptor-mediated transfer (ligand-DNA complex), electroporation, calcium phosphate precipitation or microparticle bombardment (gene gun).

Immunosuppressive drugs, such as cyclosporin, can also be administered to the patient in need to reduce the host reaction versus graft. Allografts using the cells obtained by the methods of the present invention are also useful because a single healthy donor could supply enough cells to regenerate at least partial pancreas function in multiple recipients.
Administration of a prosaposin protein product and/or modulators/effectors thereof in a pharmaceutical composition to a subject in need thereof, particularly a human patient, leads to an at least partial regeneration of pancreatic cells. Preferably, these cells are insulin producing beta-cells that will contribute to the improvement of a diabetic state. With the administration of this composition e.g. on a short term or regular basis, an increase in beta-cell mass can be achieved. This effect upon the body reverses the condition of diabetes partially or completely. As the subject's blood glucose homeostasis improves, the dosage administered may be reduced in strength. In at least some cases further administration can be discontinued entirely and the subject continues to produce a normal amount of insulin without further treatment. The subject is thereby not only treated but could be cured entirely of a diabetic condition. However, even moderate improvements in beta-cell mass can lead to a reduced requirement for exogenous insulin, improved glycemic control and a subsequent reduction in diabetic complications. In another example, the compositions of the present invention will also have efficacy for treatment of patients with other pancreatic diseases such as pancreatic cancer, dysplasia, or pancreatitis, if beta-cells are to be regenerated.

The nucleic acids of the invention or fragments thereof, may further be useful in diagnostic application, wherein the presence or amount of the nucleic acids or the proteins are to be assessed. Further antibodies that bind immunospecifically to the novel substances or the invention may be used in therapeutic or diagnostic methods.

For example, in one aspect, antibodies, which are specific for the proteins of the invention and homologous proteins, may be used directly as an effector, e.g. an antagonist or indirectly as a targeting or delivery mechanism for bringing a pharmaceutical agent to cells or tissue which express the protein. The antibodies may be generated using methods that are well known in the art. Such antibodies may include, but are not limited to, polyclonal, monoclonal, chimeric single chain, Fab fragments, and fragments produced by a Fab expression library. Neutralising antibodies, (i.e., those which inhibit dimer formation) are especially preferred for therapeutic use.

For the production of antibodies, various hosts including goats, rabbits, rats, mice, humans, and others, may be immunized by injection with the protein or any fragment or oligopeptide thereof which has immunogenic properties. Depending on the host species, various adjuvants may be used to increase immunological response. It is preferred that the peptides, fragments or oligopeptides used to induce antibodies to the protein have an amino acid sequence consisting of at least five amino acids, and more preferably at least 10 amino acids.

Monoclonal antibodies to the proteins may be prepared using any technique that provides for the production of antibody molecules by continuous cell lines in culture. These include, but are not limited to, the hybridoma technique, the human B-cell hybridoma technique, and the EBV-hybridoma technique (Köhler, G. et al. (1975) Nature 256: 495-497; Kozbor, D. et al. (1985) J. Immunol. Methods 81: 31-42; Cote, R. J. et al. Proc. Natl. Acad. Sci. 80: 2026-2030; Cole, S. P. et al. (1984) Mol. Cell Biol. 62: 109-120).

In addition, techniques developed for the production of 'chimeric antibodies', the splicing of mouse antibody genes to human antibody genes to obtain a molecule with appropriate antigen specificity and biological activity can be used (Morrison, S. L. et al. (1984) Proc. Natl. Acad. Sci. 81: 6851-6855; Neuberger, M. S. et al (1984) Nature 312: 604-608; Takeda, S. et al. (1985) Nature 314: 452-454). Alternatively, techniques described for the production of single chain antibodies may be adapted, using methods known in the art, to produce single chain antibodies specific for the proteins of the invention and homologous proteins. Antibodies with related specificity, but of distinct idiotypic composition, may be generated by chain shuffling from random combinatorial immunoglobulin libraries (Burton, D. R. (1991) Proc. Natl. Acad. Sci. 88: 11120-11123). Antibodies may also be produced by inducing in vivo production in the lymphocyte population or by screening recombinant immunoglobulin libraries or panels of highly specific binding reagents as disclosed in the literature (Orlandi, R. et al. (1989) Proc. Natl. Acad. Sci. 86: 3833-3837; Winter, G. et al. (1991) Nature 349: 293-299).

Antibody fragments, which contain specific binding sites for the proteins may also be generated. For example, such fragments include, but are not limited to, the F(ab')₂ fragments which can be produced by Pepsin digestion of the antibody molecule and the Fab fragments which can be generated by reducing the disulfide bridges of F(ab')₂ fragments. Alternatively, Fab expression libraries may be constructed to allow rapid and easy identification of monoclonal Fab fragments with the desired specificity (Huse, W. D. et al. (1989) Science 254: 1275-1281).

Various immunoassays may be used for screening to identify antibodies having the desired specificity. Numerous protocols for competitive binding and immunoradiometric assays using either polyclonal or monoclonal antibodies with established specificities are well known in the art. Such immunoassays typically involve the measurement of complex formation between the protein and its specific antibody. A two-site, monoclonal-based immunoassay utilizing monoclonal antibodies reacive to two non-interfering protein epitopes are preferred, but a competitive binding assay may also be employed (Maddox, supra).

In another embodiment of the invention, the polynucleotides or fragments thereof or nucleic acid effector molecules such as antisense molecules, aptamers, RNAi molecules or ribozymes may be used for therapeutic purposes. In one aspect, aptamers, i.e. nucleic acid molecules, which are capable of binding to a protein of the invention and modulating its activity, may be generated by a screening and selection procedure involving the use of combinatorial nucleic acid libraries.

In a further aspect, antisense molecules may be used in situations in which it would be desirable to block the transcription of the mRNA. In particular, cells may be transformed with sequences complementary to polynucleotides encoding the proteins of the invention and homologous proteins. Thus, antisense molecules may be used to modulate protein activity or to achieve regulation of gene function. Such technology is now well known in the art, and sense or antisense oligomers or larger fragments, can be designed from various locations along the coding or control regions of sequences encoding the proteins. Expression vectors derived from retroviruses, adenovirus, herpes or vaccinia viruses or from various bacterial plasmids may be used for delivery of nucleotide sequences to the targeted organ, tissue or cell population. Methods, which are well known to those skilled in the art, can be used to construct recombinant vectors, which will express antisense molecules complementary to the polynucleotides of the genes encoding the proteins of the invention and homologous proteins. These techniques are described both in Sambrook et al. (supra) and in Ausubel et al. (supra). Genes encoding the proteins of the invention and homologous proteins can be turned off by transforming a cell or tissue with expression vectors, which express high levels of polynucleotides that encode the proteins of the invention and homologous proteins or fragments thereof. Such constructs may be used to introduce untranslatable sense or antisense sequences into a cell. Even in the absence of integration into the DNA, such vectors may continue to transcribe RNA molecules until they are disabled by endogenous nucleases. Transient expression may last for a month or more with a non-replicating vector and even longer if appropriate replication elements are part of the vector system.

As mentioned above, modifications of gene expression can be obtained by designing antisense molecules, e.g. DNA, RNA or nucleic acid analogues such as PNA, to the control regions of the genes encoding the proteins of the invention and homologous proteins, i.e., the promoters, enhancers, and introns. Oligonucleotides derived from the transcription initiation site, e.g., between positions -10 and +10 from the start site, are preferred. Similarly, inhibition can be achieved using "triple helix" base-pairing methodology. Triple helix pairing is useful because it cause inhibition of the ability of the double helix to open sufficiently for the binding of polymerases, transcription factors or regulatory molecules. Recent therapeutic advances using triplex DNA have been described in the literature (Gee, J. E. et al. (1994) In; Huber, B. E. and B. I. Carr, Molecular and Immunologic Approaches, Futura Publishing Co., Mt. Kisco, N.Y.). The antisense molecules may also be designed to block translation of mRNA by preventing the transcript from binding to ribosomes.

Ribozymes, enzymatic RNA molecules, may also be used to catalyze the specific cleavage of RNA. The mechanism of ribozyme action involves sequence-specific hybridization of the ribozyme molecule to complementary target RNA, followed by endonucleolytic cleavage. Examples, which may be used, include engineered hammerhead motif ribozyme molecules that can be specifically and efficiently catalyze endonucleolytic cleavage of sequences encoding the proteins of the invention and homologous proteins. Specific ribozyme cleavage sites within any potential RNA target are initially identified by scanning the target molecule for ribozyme cleavage sites which include the following sequences: GUA, GUU, and GUC. Once identified, short RNA sequences of between 15 and 20 ribonucleotides corresponding to the region of the target gene containing the cleavage site may be evaluated for secondary structural features which may render the oligonucleotide inoperable. The suitability of candidate targets may also be evaluated by testing accessibiliiy to hybridization with complementary oligonucleotides using ribonuclease protection assays.

Nucleic acid effector/modulator molecules, e.g. antisense molecules and ribozymes of the invention may be prepared by any method known in the art for the synthesis of nucleic acid molecules. These include techniques for chemically synthesizing oligonucleotides such as solid phase phosphoramidite chemical synthesis. Alternatively, RNA molecules may be generated by in vitro and in vivo transcription of DNA sequences. Such DNA sequences may be incorporated into a variety of vectors with suitable RNA polymerase promoters such as T7 or SP6. Alternatively, these cDNA constructs that synthesize antisense RNA constitutively or inducibly can be introduced into cell lines, cells or tissues. RNA molecules may be modified to increase intracellular stability and half-life. Possible modifications include, but are not limited to, the addition of flanking sequences at the 5' and/or 3' ends of the molecule or modifications in the nucleobase, sugar and/or phosphate moieties, e.g. the use of phosphorothioate or 2' O-methyl rather than phosphodiesterase linkages within the backbone of the molecule. This concept is inherent in the production of PNAs and can be extended in all of these molecules by the inclusion of non-traditional bases such as inosine, queosine, and wybutosine, as well as acetyl-, methyl-, thio-, and similarly modified forms of adenine, cytidine, guanine, thymine, and uridine which are not as easily recognized by endogenous endonucleases.

Many methods for introducing vectors into cells or tissues are available and equally suitable for use in vivo, in vitro, and ex vivo. For ex vivo therapy, vectors may be introduced into stem cells taken from the patient and clonally propagated for autologous transplant back into that same patient. Delivery by transfection and by liposome injections may be achieved using methods, which are well known in the art. Any of the therapeutic methods described above may be applied to any suitable subject including, for example, mammals such as dogs, cats, cows, horses, rabbits, monkeys, and most preferably, humans.

An additional disclosure of the invention relates to the administration of a pharmaceutical composition, in conjunction with a pharmaceutically acceptable carrier, for any of the therapeutic effects discussed above. Such pharmaceutical compositions may consist of the nucleic acids and the proteins of the invention and homologous nucleic acids or proteins, antibodies to the proteins of the invention and homologous proteins, mimetics, agonists, antagonists or inhibitors of the proteins of the invention and homologous proteins or nucleic acids. The compositions may be administered alone or in combination with at least one other agent, such as stabilising compound, which may be administered in any sterile, biocompatible pharmaceutical carrier, including, but not limited to, saline, buffered saline, dextrose, and water. The compositions may be administered to a patient alone or in combination with other agents, drugs or hormones. The pharmaceutical compositions utilized in this invention may be administered by any number of routes including, but not limited to, oral, intravenous, intramuscular, intra-arterial, intramedullary, intrathecal, intraventricular, transdermal, subcutaneous, intraperitoneal, intranasal, enteral, topical, sublingual or rectal means.

In addition to the active ingredients, these pharmaceutical compositions may contain suitable pharmaceutically-acceptable carriers comprising excipients and auxiliaries, which facilitate processing of the active compounds into preparations, which can be used pharmaceutically. Further details on techniques for formulation and administration may be found in the latest edition of Remington's Pharmaceutical Sciences (Maack Publishing Co., Easton, Pa.).

Pharmaceutical compositions suitable for use in the invention include compositions wherein the active ingredients are contained in an effective amount to achieve the intended purpose. The determination of an effective dose is well within the capability of those skilled in the art. For any compound, the therapeutically effective dose can be estimated initially either in cell culture assays, e.g., of preadipocyte cell lines or in animal models, usually mice, rabbits, dogs or pigs. The animal model may also be used to determine the appropriate concentration range and route of administration. Such information can then be used to determine useful doses and routes for administration in humans. A therapeutically effective dose refers to that amount of active ingredient, for example the nucleic acids or the proteins of the invention or fragments thereof or antibodies, which is sufficient for treating a specific condition. Therapeutic efficacy and toxicity may be determined by standard pharmaceutical procedures in cell cultures or experimental animals, e.g., ED50 (the dose therapeutically effective in 50% of the population) and LD50 (the dose lethal to 50% of the population). The dose ratio between therapeutic and toxic effects is the therapeutic index, and it can be expressed as the ratio, LD50/ED50. Pharmaceutical compositions, which exhibit large therapeutic indices, are preferred. The data obtained from cell culture assays and animal studies is used in formulating a range of dosage for human use. The dosage contained in such compositions is preferably within a range of circulating concentrations that include the ED50 with little or no toxicity. The dosage varies within this range depending upon the dosage from employed, sensitivity of the patient, and the route of administration. The exact dosage will be determined by the practitioner, in light of factors related to the subject that requires treatment. Dosage and administration are adjusted to provide sufficient levels of the active moiety or to maintain the desired effect. Factors, which may be taken into account, include the severity of the disease state, general health of the subject, age, weight, and gender of the subject, diet, time and frequency of administration, drug combination(s), reaction sensitivities, and tolerance/response to therapy. Long-acting pharmaceutical compositions may be administered every 3 to 4 days, every week or once every two weeks depending on half-life and clearance rate of the particular formulation. Normal dosage amounts may vary from 0.1 to 100,000 µg, up to a total dose of about 1 g, depending upon the route of administration. Guidance as to particular dosages and methods of delivery is provided in the literature and generally available to practitioners in the art. Those skilled in the art employ different formulations for nucleotides than for proteins or their inhibitors. Similarly, delivery of polynucleotides or polypeptides will be specific to particular cells, conditions, locations, etc.

In another embodiment, antibodies which specifically bind to the proteins may be used for the diagnosis of conditions or diseases characterized by or associated with over- or underexpression of the proteins of the invention and homologous proteins or in assays to monitor patients being treated with the proteins of the invention and homologous proteins, or effectors thereof, e.g. agonists, antagonists, or inhibitors. Diagnostic assays include methods which utilize the antibody and a label to detect the protein in human body fluids or extracts of cells or tissues. The antibodies may be used with or without modification, and may be labeled by joining them, either covalently or non-covalently, with a reporter molecule. A wide variety of reporter molecules, which are known in the art may be used several of which are described above.

A variety of protocols including ELISA, RIA, and FACS for measuring proteins are known in the art and provide a basis for diagnosing altered or abnormal levels of gene expression. Normal or standard values for gene expression are established by combining body fluids or cell extracts taken from normal mammalian subjects, preferably human, with antibodies to the protein under conditions suitable for complex formation. The amount of standard complex formation may be quantified by various methods, but preferably by photometric means. Quantities of protein expressed in control and disease, samples e.g. from biopsied tissues are compared with the standard values. Deviation between standard and subject values establishes the parameters for diagnosing disease.

In another embodiment of the invention, the polynucleotides specific for the proteins of the invention and homologous proteins may be used for diagnostic purposes. The polynucleotides, which may be used, include oligonucleotide sequences, antisense RNA and DNA molecules, and PNAs. The polynucleotides may be used to detect and quantitate gene expression in biopsied tissues in which gene expression may be correlated with disease. The diagnostic assay may be used to distinguish between absence, presence, and excess gene expression, and to monitor regulation of protein levels during therapeutic intervention.

In one aspect, hybridization with probes which are capable of detecting polynucleotide sequences, including genomic sequences, encoding the proteins of the invention and homologous proteins or closely related molecules, may be used to identify nucleic acid sequences which encode the respective protein. The hybridization probes of the subject invention may be DNA or RNA and are preferably derived from the nucleotide sequence of the polynucleotide encoding the proteins of the invention or from a genomic sequence including, promoter, enhancer elements, and introns of the natural occurring gene. Hybridization probes may be labeled by a variety of reporter groups, for example, radionuclides such as ³²P or ³⁵S or enzymatic labels, such as alkaline phosphatase coupled to the probe via avidin/biotin coupling systems, and the like.

Polynucleotide sequences specific for the proteins of the invention and homologous nucleic acids may be used for the diagnosis of conditions or diseases, which are associated with the expression of the proteins. Examples of such conditions or diseases include, but are not limited to, metabolic diseases and disorders, including obesity, diabetes, and/or metabolic syndrome. Polynucleotide sequences specific for the proteins of the invention and homologous proteins may also be used to monitor the progress of patients receiving treatment for metabolic diseases and disorders, including obesity, diabetes, and/or metabolic syndrome. The polynucleotide sequences may be used qualitative or quantitative assays, e.g. in Southern or Northern analysis, dot blot or other membrane-based technologies; in PCR technologies; or in dip stick, pin, ELISA or chip assays utilizing fluids or tissues from patient biopsies to detect altered gene expression.

In a particular aspect, the nucleotide sequences specific for the proteins of the invention and homologous nucleic acids may be useful in assays that detect activation or induction of various metabolic diseases and disorders, including obesity, diabetes, and/or metabolic syndrome. The nucleotide sequences may be labeled by standard methods, and added to a fluid or tissue sample from a patient under conditions suitable for the formation of hybridization complexes. After a suitable incubation period, the sample is washed and the signal is quantitated and compared with a standard value. The presence of altered levels of nucleotide sequences encoding the proteins of the invention and homologous proteins in the sample indicates the presence of the associated disease. Such assays may also be used to evaluate the efficacy of a particular therapeutic treatment regimen in animal studies, in clinical trials or in monitoring the treatment of an individual patient.

In order to provide a basis for the diagnosis of a disease associated with expression of the proteins of the invention and homalogous proteins, a normal or standard profile for expression is established. This may be accomplished by combining body fluids or cell extracts taken from normal subjects, either animal or human, with a sequence or a fragment thereof, which is specific for the nucleic acids encoding the proteins of the invention and homologous nucleic acids, under conditions suitable for hybridization or amplification. Standard hybridization may be quantified by comparing the values obtained from normal subjects with those from an experiment where a known amount of a substantially purified polynucleotide is used. Standard values obtained from normal samples may be compared with values obtained from samples from patients who are symptomatic for disease. Deviation between standard and subject values is used to establish the presence of disease. Once disease is established and a treatment protocol is initiated, hybridization assays may be repeated on a regular basis to evaluate whether the level of expression in the patient begins to approximate that, which is observed in the normal patient. The results obtained from successive assays may be used to show the efficacy of treatment over a period ranging from several days to months.

With respect to metabolic diseases such as described above the presence of an unusual amount of transcript in biopsied tissue from an individual may indicate a predisposition for the development of the disease or may provide a means for detecting the disease prior to the appearance of actual clinical symptoms. A more definitive diagnosis of this type may allow health professionals to employ preventative measures or aggressive treatment earlier thereby preventing the development or further progression of the metabolic diseases and disorders.

Additional diagnostic uses for oligonucleotides designed from the sequences encoding the proteins of the invention and homologous proteins may involve the use of PCR. Such oligomers may be chemically synthesized, generated enzymatically or produced from a recombinant source. Oligomers will preferably consist of two nucleotides sequences, one with sense orientation (5prime.fwdarw.3prime) and another with antisense (3prime.rarw.5prime), employed under optimized conditions for identification of a specific gene or condition. The same two oligomers, nested sets of oligomers or even a degenerate pool of oligomers may be employed under less stringent conditions for detection and/or quantification of closely related DNA or RNA sequences.

In another embodiment of the invention, the nucleic acid sequences may also be used to generate hybridization probes, which are useful for mapping the naturally occurring genomic sequence. The sequences may be mapped to a particular chromosome or to a specific region of the chromosome using well known techniques. Such techniques include FISH, FACS or artificial chromosome constructions, such as yeast artificial chromosomes, bacterial artificial chromosomes, bacterial P1 constructions or single chromosome cDNA libraries as reviewed in Price, C. M. (1993) Blood Rev. 7: 127-134, and Trask, B. J. (1991) Trends Genet. 7:149-154. FISH (as described in Verma et al. (1988) Human Chromosomes: A Manual of Basic Techniques, Pergamon Press, New York, N.Y.). The results may be correlated with other physical chromosome mapping techniques and genetic map data. Examples of genetic map data can be found in the 1994 Genome Issue of Science (265:1981f). Correlation between the location of the gene encoding the proteins of the invention on a physical chromosomal map and a specific disease or predisposition to a specific disease, may help to delimit the region of DNA associated with that genetic disease.

The nucleotide sequences of the subject invention may be used to detect differences in gene sequences between normal, carrier or affected individuals. An analysis of polymorphisms, e.g. single nucleotide polymorphisms may be carried out. Further, in situ hybridization of chromosomal preparations and physical mapping techniques such as linkage analysis using established chromosomal markers may be used for extending genetic maps. Often the placement of a gene on the chromosome of another mammalian species, such as mouse, may reveal associated markers even if the number or arm of a particular human chromosome is not known. New sequences can be assigned to chromosomal arms or parts thereof, by physical mapping. This provides valuable information to investigators searching for disease genes using positional cloning or other gene discovery techniques. Once the disease or syndrome has been crudely localized by genetic linkage to a particular genomic region, for example, AT to 11q22-23 (Gatti, R. A. et al. (1988) Nature 336: 577-580), any sequences mapping to that area may represent associated or regulatory genes for further investigation. The nucleotide sequences of the subject invention may also be used to detect differences in the chromosomal location due to translocation, inversion, etc. among normal, carrier or affected individuals.

In another embodiment of the invention, the proteins of the invention, their catalytic or immunogenic fragments or oligopeptides thereof, an in vitro model, a genetically altered cell or animal, can be used for screening libraries of compounds in any of a variety of drug screening techniques. One can identify effectors, e.g. receptors, enzymes, proteins, ligands, or substrates that bind to, modulate or mimic the action of one or more of the proteins of the invention. The protein or fragment thereof employed in such screening may be free in solution, affixed to a solid support, borne on a cell surface, or located intracellulary. The formation of binding complexes, between the proteins of the invention and the agent tested, may be measured. Agents could also, either directly or indirectly, influence the activity of the proteins of the invention.

In addition activity of the proteins of the invention against their physiological substrate(s) or derivatives thereof could be measured in cell-based or cell-free assays. Agents may also interfere with posttranslational modifications of the protein, such as phosphorylation and dephosphorylation, farnesylation, palmitoylation, acetytation, alkylation, ubiquitination, proteolytic processing, subcellular localization and degradation. Moreover, agents could influence the dimerization or oligomerization of the proteins of the invention or, in a heterologous manner, of the proteins of the invention with other proteins, for example, but not exclusively, docking proteins, enzymes, receptors, or translation factors. Agents could also act on the physical interaction of the proteins of this invention with other proteins, which are required for protein function, for example, but not exclusively, their downstream signaling.

Methods for determining protein-protein Interaction are well known in the art. For example binding of a fluorescently labeled peptide derived from the interacting protein to the protein of the Invention, or vice versa, could be detected by a change in polarisation. In case that both binding partners, which can be either the full length proteins as well as one binding partner as the full length protein and the other just represented as a peptide are fluorescently labeled, binding could be detected by fluorescence energy transfer (FRET) from one fluorophore to the other. In addition, a variety of commercially available assay principles suitable for detection of protein-protein Interaction are well known In the art, for example but not exclusively AlphaScreen (PerkinElmer) or Scintillation Proximity Assays (SPA) by Amersham. Alternatively, the interaction of the proteins of the invention with cellular proteins could be the basis for a cell-based screening assay, in which both proteins are fluorescently labeled and interaction of both proteins is detected by analysing cotranslocation of both proteins with a cellular imaging reader, as has been developed for example, but not exclusively, by Cellomics or EvotecOAI. In all cases the two or more binding partners can be different proteins with one being the protein of the invention, or in case of dimerization and/or oligomerization the protein of the invention itself. Proteins of the invention, for which one target mechanism of interest, but not the only one, would be such protein/protein interactions are PSAP, SFTPB, and/or FLJ40379.

Of particular interest are screening assays for agents that have a low toxicity for mammalian cells. The term "agent" as used herein describes any molecule, e.g. protein or pharmaceutical, with the capability of altering or mimicking the physiological function of one or more of the proteins of the invention. Candidate agents encompass numerous chemical classes, though typically they are organic molecules, preferably small organic compounds having a molecular weight of more than 50 and less than about 2,500 Daltons. Candidate agents comprise functional groups necessary for structural interaction with proteins, particularly hydrogen bonding, and typically include at least an amine, carbonyl, hydroxyl or carboxyl group, preferably at least two of the functional chemical groups. The candidate agents often comprise carbocyclic or heterocyclic structures and/or aromatic or polyaromatic structures substituted with one or more of the above functional groups.

Candidate agents are also found among biomolecules including peptides, saccharides, fatty acids, steroids, purines, pyrimidines, nucleic acids and derivatives, structural analogs or combinations thereof. Candidate agents are obtained from a wide variety of sources including libraries of synthetic or natural compounds. For example, numerous means are available for random and directed synthesis of a wide variety of organic compounds and biomolecules, including expression of randomized oligonucleotides and oligopeptides. Alternatively, libraries of natural compounds in the form of bacterial, fungal, plant and animal extracts are available or readily produced. Additionally, natural or synthetically produced libraries and compounds are readily modified through conventional chemical, physical and biochemical means, and may be used to produce combinatorial libraries. Known pharmacological agents may be subjected to directed or random chemical modifications, such as acylation, alkylation, esterification, amidification, etc. to produce structural analogs. Where the screening assay is a binding assay, one or more of the molecules may be joined to a label, where the label can directly or indirectly provide a detectable signal.

Another technique for drug screening, which may be used, provides for high throughput screening of compounds having suitable binding affinity to the protein of interest as described in published PCT application WO84/03564. In this method, as applied to the proteins of the invention large numbers of different small test compounds, e.g. aptamers, peptides, low-molecular weight compounds etc., are provided or synthesized on a solid substrate, such as plastic pins or some other surface. The test compounds are reacted with the proteins or fragments thereof, and washed. Bound proteins are then detected by methods well knows in the art. Purified proteins can also be coated directly onto plates for use in the aforementioned drug screening techniques. Alternatively, non-neutralizing antibodies can be used to capture the peptide and immobilize it on a solid support. In another embodiment, one may use competitive drug screening assays in which neutralizing antibodies capable of binding the protein specifically compete with a test compound for binding the protein. In this manner, the antibodies can be used to detect the presence of any peptide, which shares one or more antigenic determinants with the protein.

In a further embodiment, the present invention allows the production of cells for the identification and/or characterisation of compounds which stimulate beta-cell differentiation, insulin secretion and/or glucose response, more particularly of compounds which increase the prosaposin expression level or function. This method is particularly suitable for in vivo testing for diagnostic applications and drug development or screening. The compound of interest is added to suitable cells and the prosaposin expression or function is determined. Alternatively, a compound of interest is added to a prosaposin-treated cell and the effect on cell differentiation and/or insulin production is determined. Preferably, differentiated insulin-producing cells used. Insulin levels in treated cells can be determined, e.g. quantified by Enzyme Linked Immunoabsorbent Assay (ELISA) or Radio Immune Assay (RIA). Using this method, a large number of compounds can be screened and compounds that induce prosaposin expression or support the activity of prosaposin leading to a beta-cell differentiation and/or an increase in insulin secretion can be identified readily.

In a high-throughput screening method, the cells are transfected with a DNA construct, e.g. a viral or non-viral vector containing a reporter gene, e.g. the lacZ gene or the GFP gene, under regulatory control of a promoter of a gene involved in beta-cell differentiation, e.g. preferably a Pax4 promoter. The transfected cells are divided into aliquots and each aliquot is contacted with a test substance, e.g. candidate 1, candidate 2, and candidate 3. The activity of the reporter gene corresponds to the capability of the test compound to induce beta-cell differentiation.

In a further embodiment (which may be combined with the high-throughput screening as described above) a medium throughput validation is carried out. Therein, the test compound is added to cells being cultivated and the prosaposin expression and/or the insulin production is determined. Following an initial high throughput assay, such as the cell based assay outlined above where e.g. a Pax4 promoter is used as marker for beta-cell regeneration, the activity of candidate molecules to induce beta-cell differentiation is tested in a validation assay comprising adding said compounds to the culture media of the embryoid bodies. Differentiation into insulin-producing cells is then evaluated, e.g. by comparison to wild type and/or Pax4 expressing cells to assess the effectiveness of a compound.

The nucleic acids encoding the protein of the invention can be used to generate transgenic animals or site-specific gene modifications in cell lines. These transgenic non-human animals are useful in the study of the function and regulation of the protein of the invention in vivo. Transgenic animals, particularly mammalian transgenic animals, can serve as a model system for the investigation of many developmental and cellular processes common to humans. A variety of non-human models of metabolic disorders can be used to test effectors/modulators of the protein of the invention. Misexpression (for example, overexpression or lack of expression) of the protein of the invention, particular feeding conditions, and/or administration of biologically active compounds can create models of metablic disorders.

In one embodiment of the invention, such assays use mouse models of insulin resistance and/or diabetes, such as mice carrying gene knockouts in the leptin pathway (for example, ob (leptin) or db (leptin receptor) mice). Such mice develop typical symptoms of diabetes, show hepatic lipid accumulation and frequently have increased plasma lipid levels (see Bruning J.C. et al., 1998, supra). Susceptible wild type mice (for example C57BI/6) show similiair symptoms if fed a high fat diet. In addition to testing the expression of the proteins of the invention in such mouse strains (see Examples section), these mice could be used to test whether administration of a candidate effector/modulator alters for example lipid accumulation in the liver, in plasma, or adipose tissues using standard assays well known in the art, such as FPLC, colorimetric assays, blood glucose level tests, insulin tolerance tests and others.

Transgenic animals may be made through homologous recombination in non-human embryonic stem cells, where the normal locus of the gene encoding the protein of the invention is altered. Alternatively, a nucleic acid construct encoding the protein of the invention is injected into oocytes and is randomly integrated into the genome. Vectors for stable integration include plasmids, retroviruses and other animal viruses, yeast artificial chromosomes (YACs), and the like. The modified cells or animals are useful in the study of the function and regulation of the protein of the invention. For example, a series of small deletions and/or substitutions may be made in the gene that encodes the protein of the invention to determine the role of particular domains of the protein, functions in pancreatic differentiation, etc.

Furthermore, variants of the gene of the invention like specific constructs of interest include anti-sense molecules, which will block the expression of the protein of the invention, or expression of dominant negative mutations. A detectable marker, such as for example lac-Z or luciferase may be introduced in the locus of the gene of the invention, where up regulation of expression of the gene of the invention will result in an easily detected change in phenotype.

One may also provide for expression of the gene of the invention or variants thereof in cells or tissues where it is not normally expressed or at abnormal times of development. In addition, by providing expression of the protein of the invention in cells in which they are not normally produced, one can induce changes in cell behavior.

DNA constructs for homologous recombination will comprise at least portions of the gene of the invention with the desired genetic modification, and will include regions of homology to the target locus. DNA constructs for random integration do not need to contain regions of homology to mediate recombination. Conveniently, markers for positive and negative selection are included. DNA constructs for random integration will consist of the nucleic acids encoding the protein of the invention, a regulatory element (promoter), an intron and a poly-adenylation signal. Methods for generating cells having targeted gene modifications through homologous recombination are known in the art. For non-human embryonic stem (ES) cells, an ES cell line may be employed, or embryonic cells may be obtained freshly from a host, e.g. mouse, rat, guinea pig, etc. Such cells are grown on an appropriate fibroblast-feeder layer and are grown in the presence of leukemia inhibiting factor (LIF).

When non-human ES or embryonic cells or somatic pluripotent stem cells have been transfected, they may be used to produce transgenic animals. After transfection, the cells are plated onto a feeder layer in an appropriate medium. Cells containing the construct may be selected by employing a selective medium. After sufficient time for colonies to grow, they are picked and analyzed for the occurrence of homologous recombination or integration of the construct. Those colonies that are positive may then be used for embryo transfection and morula aggregation. Briefly, morulae are obtained from 4 to 6 week old superovulated females, the Zona Pellucida is removed and the morulae are put into small depressions of a tissue culture dish. The ES cells are trypsinized, and the modified cells are placed into the depression closely to the morulae. On the following day the aggregates are transfered into the uterine horns of pseudopregnant females. Females are then allowed to go to term. Chimeric offsprings can be readily detected by a change in coat color and are subsequently screened for the transmission of the mutation into the next generation (F1-generation). Offspring of the F1-generation are screened for the presence of the modified gene and males and females having the modification are mated to produce homozygous progeny. If the gene alterations cause lethality at some point in development, tissues or organs can be maintained as allogenic or congenic grafts or transplants, or in vitro culture. The transgenic animals may be any non-human mammal, such as laboratory animal, domestic animals, etc., for example, mouse, rat, guinea pig, sheep, cow, pig, and others. The transgenic animals may be used in functional studies, drug screening, and other applications and are useful in the study of the function and regulation of the protein of the invention in vivo.

Finally, the application also discloses a kit comprising at least one of
(a) a nucleic add molecule coding for a protein of the invention or a fragment thereof;
(b) a protein of the invention or a fragment or an isoform thereof;
(c) a vector comprising the nucleic acid of (a);
(d) a host cell comprising the nucleic acid of (a) or the vector of (c);
(e) a polypeptide encoded by the nucleic acid of (a);
(f) a fusion polypeptide encoded by the nucleic acid of (a);
(g) an antibody, an aptamer or another modulator/effector of the nucleic acid of (a) or the polypeptide of (b), (e) or (f) and
(h) an anti-sense oligonucleotide of the nucleic acid of (a).

The kit may be used for diagnostic or therapeutic purposes or for screening applications as described above. The kit may further contain user instructions.

The Figures show:
**Figure 1** shows the content of energy storage metabolites (ESM; triglyceride (TG) and glycogen) of Drosophila Saposin-related (GadFly Accession Number CG12070) mutants. Shown is the change of triglyceride content of HD-EP(3) 36824 flies caused by integration of the P-vector into the annotated transcription unit ('HD-36824 (TG, 70°C)', column 3 and 'HD-36824 (TG, 90° C)', column 6) in comparison to controls containing about 880 fly lines of the proprietary EP collection ('HD-control (TG, 70°C)'), column 1) and wild-type controls determined in 4 independent assays (referred to as 'WT-control (TG, 70°C)', column 2), and in comparison to controls containing about 2100 fly lines of the proprietary EP collection ('HD-control (TG, 90°C)'), (column 4) and wild-type controls determined in more than 80 independent assays (referred to as 'WT-control (TG, 90°C)', column 5). Also shown is the change of glycogen content of HD-EP(3)36824 flies caused by integration of the P-vector the into the annotated transcription unit ('HD-36824 (glycogen, 90°C)', column 8) in comparison to an internal assay control including two wild-type strains and a one HD-line (referred to as 'assay control (glycogen, 90°C)' column 7).
**Figure 2** shows the molecular organization of the mutated Drosophila Saposin-related gene locus (referred to as Sap-r; GadFly Accession Number CG12070).
**Figure 3** shows human saposin-related proteins: Prosaposin (variant Gaucher disease and variant metachromatic leukodystrophy, PSAP), human surfactant, pulmonary-associated protein B (SFTPB), and human hypothetical protein FLJ40379.
   **Figure 3A** shows the nucleic acid sequence of human PSAP (SEQ ID NO: 1).
   **Figure 3B** shows the amino acid sequence (one-letter code) of human PSAP (SEQ ID NO: 2).
   **Figure 3C** shows the nucleic acid sequence of human SFTPB (SEQ ID NO: 3).
   **Figure 3D** shows the amino acid sequence (one-letter code) of human SFTPB (SEQ ID NO: 4).
   **Figure 3E** shows the nucleic acid sequence of human FLJ40379 (SEQ ID NO: 5).
   **Figure 3F** shows the amino acid sequence (one-letter code) of human FLJ40379 (SEQ ID NO: 6).
   **Figure 3G** shows the comparison (ClustalW (1.83) protein sequence alignment analysis) of human and Drosophila proteins. Gaps in the alignment are represented as -. In the figure 'PSAP Hs' refers to human prosaposin, 'FLJ40379 Hs' refers to human hypothetical protein FLJ40379, 'SFTPB Hs' refers to human surfactant, pulmonary-associated protein B, and 'Sap-r Dm' refers to Drosophila Saposin-related protein.
**Figure 4** shows the quantitative analysis of Sap-r homolog prosaposin (Psap) expression in mammalian tissues. The relative RNA-expression is shown on the Y-axis, in Figure 4A to 4C the tissues tested are given on the X-axis. WAT refers to white adipose tissue, BAT refers to brown adipose tissue. In Figure 4D, the X-axis represents the time axis. 'd0' refers to day 0 (start of the experiment), 'd2' - 'd10' refers to day 2 - day 10 of adipocyte differentiation).
   **Figure 4A** shows the quantitative analysis of Psap expression in mouse wild-type tissues.
   **Figure 4B** shows the quantitative analysis of Psap expression in wild-type mice (wt-mice), compared to genetically obese mice (ob/ob-mice) and to fasted mice (fasted-mice).
   **Figure 4C** shows the quantitative analysis of Psap expression in mice fed with a control diet compared to mice fed with a high fat diet.
   **Figure 4D** shows the quantitative analysis of Psap expression in mammalian fibroblast (3T3-L1) cells, during the differentiation from preadipocytes to mature adipocytes.
**Figure 5** shows the analysis of prosaposin (PSAP) expression in human adipocytes.
   **Figure 5A** shows the expression of PSAP in human primary abdominal adipocyte cells, during the differentiation from preadipocytes to mature adipocytes.
   **Figure 5B** shows the expression of PSAP in human SGBS cells, during the differentiation from preadipocytes to mature adipocytes.
**Figure 6** shows the prosaposin dependent induction of the differentiation of insulin producing cells.

Mouse embryonic stem (ES) cells were differentiated as described previously (patent application PCT/EP02/04362, published as WO 02/086107, which is incorporated herein by reference). At the end of the differentiation procedure, cells were harvested and total RNA was isolated. The abundance of insulin mRNA (Figure 6A) and of beta-cell glucose transporter (Glut2) mRNA (Figure 6B) was determined using quantitative RT-PCR in an Applied Biosystems, 7000 sequence detection device. Levels were normalized using 18S DNA as control and a cycle number of 36 as reference. The numbers on the vertical line refer to the abundance of the indicated transcripts relative to an abundance for which 36 cycles are necessary for detection. 'R1' refers to unmodified mouse R1 embryonic stem (ES) cells; 'Pax4' refers to R1 mouse embryonic stem (ES) cells stably transfected with a CMV-Pax4 expression construct; 'insulin expression rel. to ΔCt36' refers to expression of insulin in Figure 6A and 'Glut2 expression rel. to ΔCt36' refers to expression of beta-cell glucose transporter Glut2 in Figure 6B; ,ES' refers to mouse embryonic stem cells, as described in Example 7; 'control 293 cells' refers to the differentiation protocol as described in Example 8, with the addition of supernatant of 293 cells without prosaposin; ,Psap' refers to the differentiation protocol as described in Example 9, with the addition of prosaposin enriched supernatant of 293 cells to differentiated cells).

The examples illustrate the invention:

### Example 1: Measurement of energy storage metabolites (ESM) contents in Drosophila

Mutant flies are obtained from a fly mutation stock collection. The flies are grown under standard conditions known to those skilled in the art. In the course of the experiment, additional feedings with bakers yeast (Saccharomyces cerevisiae) are provided for the EP-line HD-EP(3)36824. The average change of triglyceride and glycogen (herein referred to as energy storage metabolites, ESM) content of Drosophila containing the EP-vectors as homozygous viable integration was investigated in comparison to control flies, respectively (see Figure 1). For determination of ESM content, flies were incubated for 5 min at 70°C or 90°C in an aqueous buffer using a waterbath, followed by hot extraction. After another 5 min incubation at 70°C or 90°C and mild centrifugation, the triglyceride content of the flies extract was determined using Sigma Triglyceride (INT 336-10 or -20) assay by measuring changes in the optical density according to the manufacturer's protocol, and the glycogen content of the flies extract was determined using Roche (Starch UV-method Cat. No. 0207748) assay by measuring changes in the optical density according to the manufacturer's protocol. As a reference the protein content of the same extract was measured using BIO-RAD DC Protein Assay according to the manufacturer's protocol. These experiments and assays were repeated several times.

The average triglyceride level of 883 fly lines of the proprietary EP-collection determined at 70°C (referred to as 'HD-control (TG, 70°C)') is shown as 100% in the first column in Figure 1. The average triglyceride level of Drosophila wild-type strain Oregon R flies determined in 4 independent assays at 70°C (referred to as 'WT-control (TG, 70°C)') is shown as 116% in the second column in Figure 1. The average triglyceride level (µg triglyceride/µg protein) of 2108 fly lines of the proprietary EP-collection determined at 90°C (referred to as 'HD-control (TG, 90°C)') is shown as 100% in the fourth column in Figure 1. The average triglyceride level (µg triglyceride/µg protein) of Drosophila wild-type strain Oregon R flies determined in 84 independent assays at 90°C (referred to as 'WT-control (TG, 90°C)') is shown as 102% in the fifth column of Figure 1. The average glycogen level (µg glycogen/µg protein) of an internal assay control consisting of two different wild-type strains and an inconspicuous EP-line of the HD stock collection (referred to as 'assay control (glycogen, 90° C)') is shown as 100% in the seventh column in Figure 1. The average triglyceride level (µg triglyceride/µg protein) of all flies of the EP collection (referred to as 'EP-control') is shown as 100% in the first column in Figure 5. Standard deviations of the measurements are shown as thin bars.

HD-EP(3)36824 homozygous flies show constantly a lower triglyceride content than the controls (column 3 in Figure 1, 'HD-36824 (TG, 70°C)'; column 6 in Figure 1, 'HD-36824 (TG, 90°C)'). HD-EP(2)21554 homozygous flies also show a lower glycogen content than the controls (column 8 in Figure 1, 'HD-36824 (glycogen, 90°C)'). Therefore, the loss of gene activity is responsible for changes in the metabolism of the energy storage metabolites.

### Example 2: Identification of Drosophila genes responsible for changes in metabolite contents

Genomic DNA sequences were isolated that are localized directly adjacent to the EP vector (herein HD-EP(3)36824) integration. Using the isolated genomic sequences from HD-EP(3)36824 homozygous flies public databases like Berkeley Drosophila Genome Project (GadFly) were screened. The chromosomal localization site of HD-EP(3)36824 vector integration is at gene locus 3R, 100A6-7 (Flybase and Gadfly). The homozygous viable integration site of the HD-EP(3)36824 vector into base pair 535 of the Sap-r transcript CG12070-RA and 61 base pairs 5prime of the Sap-r transcript CG12070-RB in antisense orientation was confirmed. Therefore, expression of the cDNAs encoded by Sap-r could be affected by integration of the vector of line HD-EP (3)36824 leading to a change in the amount of energy storage metabolites. Figure 2 shows the molecular organization of this gene locus. A black double arrow in middle of the Figure represents the genomic DNA sequence. The space between two ticks represents a stretch of 1000 base pairs. The black triangle labeled 'HD-EP36824' indicates the integration site of the EP-vector. The transcripts of Sap-r (as predicted by the Berkeley Drosophila Genome Project) are shown as dark gray bars (exons) linked by dark gray lines (introns) in the lower half of the Figure labeled as 'Sap-r'.

**Table 1** is summarizing the data of our molecular analysis of the Drosophila proteins identified in this invention as being involved in the regulation of the metabolism.

**Table 1. Molecular analysis of Drosophila Saposin-related**

| **Analysis** | **Result** |
|---|---|
| **Protein domains** | Beta-Ig-H3/Fasciclin (Flybase) |
| **InterFro analysis** | Saposin type B, Saposin type A, Surfactant protein B |
| **Locus** | 3R, 100A6-7 (Flybase, Gadfly release 3) |
| **cDNA (sap-r)** | AI108030 (578 base pairs mRNA), AI109190 (635 base pairs mRNA) |
| **genomic DNA** | AE003775 |
| **RefSeq (Sap-r)** | NM 079858, NM 170529, NP 524597, NP 73340S |
| **Drosophila mutations & mutants** | Homozygotes for deficiencies removing Sap-r are fertile and viable and show no obvious phenotype. (Flybase) |

### Example 3: Identification of the human Saposin-related homologous proteins

Saposin-related homologous proteins and nucleic acid molecules coding therefore are obtainable from insect or vertebrate species, e.g. mammals or birds, preferably from humans, mouse, or Drosophila. Sequences homologous to Drosophila Saposin-related protein were identified using the publicly available program BLASTP 2.2.3 of the non-redundant protein data base of the National Center for Biotechnology Information (NCBI) (see, Altschul et al., 1997, Nucleic Acids Res. 25:3389-3402). Table 2 shows preferred human homologs of the Drosophila Saposin-related gene.

The term "polynucleotide comprising the nucleotide sequence as shown in GenBank Accession number" relates to the expressible gene of the nucleotide sequences deposited under the corresponding GenBank Accession number. The term "GenBank Accession number" relates to NCBI GenBank database entries (Ref.: Benson et al., Nucleic Acids Res. 28 (2000) 15-18).

**Table 2. Human proteins homologous to Drosophila Saposin-related proteins**

| |
|---|
| **I. Human proteins homologous to Drosonhila Sap-r-** |
| |
| **IA. Human prosaposin (PSAP)** |
| NCBI human locus identification (ID): 5660, Hs PSAP, prosaposin (variant Gaucher disease and variant metachromatic leukodystrophy), 10q21-q22 |
| RefSeq: GenBank Accession Number: NM_002778 |
| |
| **IB. Human surfactant, pulmonary-associated protein B (SFTPB)** |
| NCBI human locus identification (ID): 6439; Hs SFTPB, surfactant, pulmonary-associated protein B, 2p12-p11.2 |
| Aliases: SP-B, PSP-B, SFTB3, SFTP3 |
| RefSeq: GenBank Accession Number: NM_000542 |
| |
| **IC. Human hypothetical protein FLJ40379** |
| Nucleotide: ENSEMBL Accession Number: ENSG00000173005 |
| Protein: ENSEMBL Accession Number: ENSP00000308224, International Protein Index (IPI) Accession Number: IPI00163920 |

### Example 4: Identification of secreted factors expressed in pancreas

A screen for secreted factors expressed in developing mouse pancreas was carried out according to methods known by those skilled in the art (see, for example Pera E.M. and De Robertis E.M., (2000) Mech Dev 96(2): 183-195) with several modifications.

### Expression cDNA library:

During organogenesis, the pancreatic bud is surrounded and influenced by the associated mesenchyme. (see for example, Madsen O.D. et al., (1996) Eur. J. Biochem. 242: 435-445 and Slack, J.M., (1995) Development 121: 1569-1580). Recently, it was suggested, that white adipocytes origin directly from mesenchymal cells (Atanossova P.K., (2003) Folia Med. 45: 41-45). During embryogenesis, the innervation and vascularization of the pancreas can be observed. Therefore, the tissue used in the screen might have contained besides pancreatic cells some adipocyte precursors, blood vessels, as well as neuronal cells.

A mouse embryonic stage 9.5-15 pancreatic bud library was prepared in pCMVSPORT-6 vector using SUPERSCRIPT Plasmid System from Invitrogen (cast.#18248) according to the manufacturer's instructions. The non-amplified library was electroporated into MaxEff DH10B cells (Invitrogen).

### Secretion cloning

Bacterial clones from agar plates were picked with sterile toothpicks and cultured in 96-deep-well microtiter plates in 1 ml of LB-ampicillin (see Sambrook et al., supra). Aliquots of 8 cultures were pooled, and plasmid DNAs isolated by BioRobot_9600 (QIAGEN) using QIAprep(r) Turbo BioRobot Kit (QIAGEN). Human 293 cells were cultured in 75 ml tissue culture flasks in DMEM and 10% fetal calf serum. At 90-99% confluence, the cells were splitted at 1:3 ratio and plated at poly-D-lysin (Sigma) coated 96-well plates, 150 µl/well. Next day the cells were transfected with 100-500 ng plasmids using lipofectamine 2000 (Invitrogen), 0,5 µl/well, in 100 µl/well Optimem (Invitrogen). After 6 h the medium was exchanged for fresh complete growth medium. 24 hours after transfection, the cells were washed twice with DMEM w/o Cysteine and Methionine (Invitrogen), supplemented with 1% dialysed Bovine serum (Sigma) with 50 mg/ml Heparin (Sigma) and glutamine. The cells were labeled in 50 µl/well of the same medium and 0,75 µl/well (-S35 Met-label (HARTMANN ANALYTIC GmbH, #44138). 12 hours later, 10 µl aliquots of the supernatants were harvested in 96-well PCR plates and subjected to SDS gel electrophoresis in precast 420% gradient polyacrylamide Criterion gels (Biorad) under reducing conditions, using Criterion Dodeca Cell gel running chamber (Biorad). The gels were fixed in 10% acetic acid, 25% isopropanol for 30 min, soaked 15-30 min in AMPLIFY reagent (Amersham), dried and exposed to X-OMAT (AR) film (Kodak). Positive clones were identified by sub-selection. The 8 individual bacterial clones of each positive pool were regrown in 96-well-plates, DNA of individual clones was prepared and used for transfection as described. If one of the clones yielded proteins of the same size as that of the original pool, a positive clone was identified. Positive clones were partially sequenced from the 5' end (SEQLAB, Goettingen). Sequences (of about 500 nucléotides) were compared to public nucleotide and protein databases to reveal similarity to previously described proteins.

### Example 5: Expression of the polypeptides in mammalian tissues

To analyse the expression of the polypeptides disclosed in this invention in mammalian tissues, several mouse strains (preferably mice strains C57BI/6J, C57BI/6 ob/ob and C57BI/KS db/db which are standard model systems in obesity and diabetes research) were purchased from Harlan Winkelmann (33178 Borchen, Germany) and maintained under constant temperature (preferably 22°C), 40 per cent humidity and a light / dark cycle of preferably 14/10 hours. The mice were fed a standard chow (for example, from ssniff Spezialitäten GmbH, order number ssniff M-Z V1126-000). For the fasting experiment ("fasted wild-type mice"), wild-type mice were starved for 48 h without food, but only water supplied ad libitum (see, for example, Schnetzler et al. (1993) J Clin Invest 92: 272-280, Mizuno et al., (1996) Proc Natl Acad Sci USA 93: 3434-3438). Animals were sacrificed at an age of 6 to 8 weeks. The animal tissues were isolated according to standard procedures known to those skilled in the art, snap frozen in liquid nitrogen and stored at -80°C until needed.

For analyzing the role of the proteins disclosed in this invention in the in vitro differentiation of different mammalian cell culture cells for the conversion of pre-adipocytes to adipocytes, mammalian fibroblast (3T3-L1) cells (e.g., Green & Kehinde, Cell 1: 113-116, 1974) were obtained from the American Tissue Culture Collection (ATCC, Hanassas, VA, USA; ATCC- CL 173). 3T3-L1 cells were maintained as fibroblasts and differentiated into adipocytes as described in the prior art (e.g., Qiu. et al., J. Biol. Chem. 276: 11988-11995, 2001; Slieker et al., BBRC 251: 225-229, 1998). In brief, cells were plated in DMEM/10% FCS (Invitrogen, Karlsruhe, Germany) at 50,000 cells/well in duplicates in 6-well plastic dishes and cultured in a humidified atmosphere of 5% CO₂ at 37° C. At confluence (defined as day 0: d0) cells were transferred to serum-free (SF) medium, containing DMEM/HamF12 (3:1; invitrogen), fetuin (300 µg/m); Sigma, Munich, Germany), transferrin (2 µg/ml; Sigma), pantothenate (17 µM; Sigma), biotin (1 µM; Sigma), and EGF (0.8 nM; Hoffmann-La Roche, Basel, Switzerland). Differentiation was induced by adding dexamethssone (DEX; 1 µM; Sigma), 3-methyl-isobutyl-1-methylxanthine (MIX; 0.5 mM; Sigma), and bovine insulin (5 µg/m); Invitrogen). Four days after confluence (d4), cells were kept in SF medium, containing bovine insulin (5 µg/ml) until differentiation was completed. At various time points of the differentiation procedure, beginning with day 0 (day of confluence) and day 2 (hormone addition; for example, dexamethasone and 3-isobutyl-1-methylxanthine), up to 10 days of differentiation, suitable aliquots of cells were taken every two days.

RNA was isolated from mouse tissues or cell culture cells using Trizol Reagent (for example, from Invitrogen, Karlsruhe, Germany) and further purified with the RNeasy Kit (for example, from Qiagen, Germany) in combination with an DNase-treatment according to the instructions of the manufacturers and as known to those skilled in the art. Total RNA was reverse transcribed (preferably using Superscript II RNaseH- Reverse Transcriptase, from Invitrogen, Karlsruhe, Germany) and subjected to Taqman analysis preferably using the Taqman 2xPCR Master Mix (from Applied Biosystems, Weiterstadt, Germany; the Mix contains according to the Manufacturer for example AmpliTaq Gold DNA Polymerase, AmpErase UNG, dNTPs with dUTP, passive reference Rox and optimized buffer components) on a GeneAmp 5700 Sequence Detection System (from Applied Biosystems, Weiterstadt, Germany).

### Example 6. Analysis of the differential expression of transcripts of the proteins of the invention in human tissues

RNA preparation from human primary adipose tissues and a human adipocyte cell line (SGBS) was done as described in Example 5. The target preparation, hybridization, and scanning was performed as described in the manufactures manual (see Affymetrix Technical Manual, 2002, obtained from Affmetrix, Santa Clara, USA).

The expression analysis, (using Affynetrix GeneChips) of the prosaposin (PSAP) gene using primary human abdominal adipocycte and SGBS cell differentiation clearly shows differential expression of human PSAP in adipocytes. Several independent experiments were done. All experiments show that the PSAP transcripts are the most abundant at day 12 compared to day 0 during differentiation. These data further confirm the mouse 3T3L-1 differentiation data.

Thus, the PSAP protein has to be significantly increased in order for the preadipocyctes to differentiate into mature adipocyctes. The PSAP protein in preadipocyctes has the potential to enhance adipose differentiation. Therefore, the PSAP protein might play an essential role in the regulation of human metabolism, in particular in the regulation of adipogenesis and thus it might play an essential role in obesity, diabetes, and/or metabolic syndrome.

### Example 7: Generation of ES cells expressing the Pax4 gene.

Mouse R1 ES cells (Nagy et al., (1993) Proc. Natl. Acad. Sci. USA 90: 8424-8428) were electroporated with the Pax4 gene under the control of the CMV promoter and the neomycin resistance gene under the control of the phosphoglycerate kinase I promoter (pGK-1).

ES cells were cultured in Dulbecco's modified Eagle's medium containing 4.5 g/l glucose, 10-4 M beta-mercaptoethanol, 2 nM glutamine, 1% non-essential amino acids, 1 nM Na-pyruvate, 20% FCS and 500 U/ml leukaemia inhibitory factor (LIF). Briefly, approximately 10⁷ ES cells resuspended in 0.8 ml phosphate buffered saline (PBS) were subjected to electroporation with 25 □g/ml of linearized expression vector (Joyner, Gene Targeting: A Practical Approach, Oxford University Press, New York, 1993). Five minutes after electroporation, ES cells were plated on petri dishes containing fibroblastic feeder cells previously inactivated by treatment with 100 µg/ml mitomycin C. One day after electroporation, culture medium was changed to medium containing 450 µg/ml G418. Resistant clones were separately isolated and cultured 14 days after applying the selection medium. Cells were always cultured at 37°C, 5% CO₂. These untreated and undifferentiated ES cells were used as control the experiment shown in Figure 6 (refered to as ,ES' in Figure 6).

### Example 8: Differentiation of ES cells into insulin-producing cells (refered to as 'control 293 cells' in Figure 6)

The ES cell line R1 (wild type, 'R1' in Figure 6) and ES cells constitutively expressing Pax4 (,Pax4' in Figure 6) were cultivated as embryoid bodies (EB) by the hanging drop method, as described in patent application PCT/EP02/04362, published as WO 02/086107 , with media as described below and in Table 3. The embryoid bodies were allowed to form in hanging drop cultures for 2 days and then transferred for three days to suspension cultures in petri dishes. At day 5, EBs were plated separately onto gelatin-coated 6 cm cell culture dishes containing a differentiation medium prepared with a base of Iscove modified Dulbecco's medium. After dissociation and replating at day 14 cells were cultured up to 40 days in the differentiation medium prepared with a base of Dulbecco's modified Eagle's medium: Nutrient Mixture F-12 (DMEM/F12).

### Example 9: Expression of pancreas specific genes after differentiation of ES cells into insulin-producing cells

Expression levels of pancreas specific genes was measured by Taqman analysis as described in Example 5. Total RNA was isolated from undifferentiated R1 and Pax4⁺ ES cells (control ES cells) at day 0 and differentiated R1 ES and Pax4⁺ ES cells at day 40. RNA preparation was done as described in Example 5 without using Trizol reagent.

Results show that markers for beta-cell differentiation function were expressed at higher levels in Pax4⁺ differentiated ES cells than in differentiated wild type ES cells demonstrating that activation of a pancreatic developmental control gene renders differentiation more efficient than for wild type ES cells (Figure 6). Expression of Glut2 in differentiated stem cells indicates that hormone-producing cells are capable of responding to glucose. Expression of substantial amounts of insulin in differentiated stem cells indicates that differentiated cells show a phenotype similar to beta-cells.

### Example 10: Induction of differentiation of insulin-producing cells by prosaposin (refered to as 'Psap' in Figure 6)

In order to study the effect of prosaposin to induce beta-cell differentiation in vitro, stable mouse embryonic stem (ES) cells expressing the Pax4 under the control of the cytomegalovirus (CMV) early promoter/enhancer region were generated as described in Example 7. Pax4 and wild type ES cells were then cultured in hanging drops or spinner cultures to allow the formation of embryoid bodies. Embryoid bodies were subsequently plated, enzymatically dissociated, and replated. After dissociation, cells were cultured in a differentiation medium containing various growth factors (see Table 3 for more detail). Additionally prosaposin enriched supernatant of 293 cells was added every second day until day 40. Under such conditions, the expression of insulin was significantly induced by prosaposin (Figure 6A). In addition, the addition of prosaposin to the differentiation medium did enhance the expression of the glucose transporter Glut2 (Figure 6B). By comparison, wild type ES cells did contain only very small numbers of insulin- and Glut2 producing cells at the same stage. These data demonstrate that prosaposin can significantly promote and enhance ES cells differentiation into insulin-producing cells compared to wild type ES cells.

The results shown in Figure 6 clearly demonstrate a significant induction of the differentiation of insulin-producing (Figure 6A) and glucose responsive (Figure 6B) cells, if prosaposin is added on later stages of differentiation. Thus, prosaposin has a strong inductive effect on the differentiation of insulin-producing beta cells.

**Table 3. Protocol for the induction of differentiation of insulin-producing cells by prosaposin (Psap)**

| Media B2 and B27 are described in Rolletschek et al., (2001) Mech. Dev, 105: 93-104. | | | |
|---|---|---|---|
| **Day** | **Stage of Cultivation** | **Medium** | **Coating and Analysis** |
| 0 | hanging drops (600 cells/drop) | Iscove + 20% FCS | RNA (ES cells) |
| 1 | | | |
| 2 | EBs in suspension | Iscove + 20% FCS | |
| 3 | | | |
| 4 | | | |
| 5 | plating of EBs | Iscove + 20% FCS | gelatin coating |
| +1 | | | ornithine / laminin coating |
| +2 | | | |
| +3 | | | |
| +4 | | | |
| +5 | | | |
| +6 | | | |
| +7 | | | |
| +8 | | | |
| +9 | dissociation | B2+B27+NA+10% FCS RNA (1x6 cm dish) | |
| +10 | medium change | B2+B27+NA + Psap, 50 ng/ml | |
| +11 | | | |
| +12 | medium change | + Psap, 50 ng/ml | |
| +13 | | | |
| +14 | medium change | + Psap, 50 ng/ml | |
| +15 | | | |
| +16 | medium changed | + Psap, 50 ng/ml | |
| +17 | | | |
| +18 | medium change | + Psap, 50 ng/ml | |
| +19 | | | |
| +20 | medium change | + Psap, 50 ng/ml | |
| +21 | | | |
| +22 | medium change | + Psap, 50 ng/ml | |
| +23 | | | |
| +24 | medium change | + Psap, 50 ng/ml | |
| +25 | | | |
| +26 | medium change | + Psap, 50 ng/ml | |
| +27 | | | |
| +28 | medium change | + Psap, 50 ng/ml | |
| +29 | | | |
| +30 | medium change | + Psap, 50 ng/ml | |
| +31 | | | |
| +32 | medium change | + Psap, 50 ng/ml | RNA |

### Example 11: Functional characterisation of the differentiated insulin-producing cells

One important property of beta-cells is glucose responsive insulin secretion. To test whether the Pax4 derived insulin-producing cells possessed this glucose responsive property, an *in vitro* glucose responsive assay can be performed on the differentiated cells. On the day of the assay, the differentiation medium of 12 or 6 well plate is removed and the cells are washed 3 times with Krebs Ringer Bicarbonate Hepes Bluffer (KRBH; 125 mM NaCl, 4.74 mM KCl, 1 mM CaCl₂, 1.2 mM KH₂PO₄, 1.2 mM MgSO₄ 5 mM NaHCOs, 25 mM Hepes, pH 7,4 and 0,1% BSA) supplemented with 2,8 mM Glucose. For Preincubation cells were incubated in KRBH + 2,8 mM Glucose for 2 hours at 37°C. Afterwards cells were incubated in 500 ml KRBH + 2,8 mM Glucose for 1 hour and the supernatant is then kept for measurement of basal insulin secretion. For the stimulated insulin release 500 ml KRBH containing 27.7 mM glucose is added to the cells. After 1 hour incubation at 37°C, the KRBH is recovered for measurement of glucose-induced insulin secretion and the cells were extracted with acid-ethanol. (see also Irminger, J.-C. et al., 2003, Endocrinology 144: 1368-1379). Insulin levels can be determined by an Enzyme-Linked Immunosorbent Assay (ELISA) for mouse insulin (Mercodia) and performed according to the manufacturer's recommendations.

### Example 12: Transplantation of Pax4 ES derived insulin-producing cells in STZ diabetic mice

The therapeutic potential of prosaposin induced insulin-producing cells to improve and cure diabetes can be investigated by transplanting the cells into streptozotocin induced diabetic mice. Streptozotocin is an antibiotic which is cytotoxic to beta-cells when administered at certain dosage (see Rodrigues et al.: Streptozotocin-induced diabetes, in McNeill (ed) Experimental Models of Diabetes, CRC Press LLC, 1999). Its effect is rapid, rendering an animal severely diabetic within 48 hours.

Non-fasted Male BalbC mice can be treated with STZ to develop hyperglycaemia after STZ treatment. Mice are considered diabetic if they have a blood glucose level above 10 mmol/I for more than 3 consecutive days. Cells are transplanted under the kidney capsule and into the spleen of animals. The presence of the insulin-producing cells can be confirmed by immunohistological analysis of the transplanted tissue. Results are expected to demonstrate that the transplanted cells can normalise blood glucose in diabetic animals.

Various modifications and variations of the described method and system of the invention will be apparent to those skilled in the art without departing from the scope of the invention.

## Claims

1. A saposin-related protein or nucleic acid for use in the protection and/or regeneration of beta cells in patients suffering from diabetes type I or type II.

2. The saposin-related protein or nucleic acid for use according to claim 1 in combination with another pharmaceutical composition useful to treat beta-cell degeneration.

3. The saposin-related protein or nucleic acid for use according to claim 2, wherein said other pharmaceutical composition comprises hormones, growth factors or immune modulating agents.

4. In vitro use of a saposin-related protein or nucleic acid for stimulating and/or inducing the differentiation of insulin producing cells from progenitor cells.

5. The use of claim 4, wherein the differentiation of progenitor, e.g. stem cells into insulin-producing cells in vitro comprises
(a) optionally activating one or more pancreatic genes in progenitor cells,
(b) optionally aggregating said cells to form embryoid bodies,
(c) cultivating said cells or embryoid bodies in specific differentiation media containing saposin-related protein product and
(d) identifying and optionally selecting insulin-producing cells.

6. Use of a nucleic acid molecule encoding saposin-related polypeptide or a homologue thereof or use of a polypeptide encoded thereby, or use of a fragment of said nucleic acid molecule or said polypeptide or use of a polynucleotide sequence capable of hybridizing to nucleotide sequences encoding saposin-related polypeptides or a homologue thereof, or use of alleles of the genes encoding said polypeptides, or use of nucleic acid sequences encoding said polypeptides, wherein said nucleic acid sequences carry deletions, insertions or substitutions of different nucleotides resulting in a polynucleotide encoding the same or a functionally equivalent polypeptide, or use of said encoded polypeptide, wherein the polypeptide contains deletions insertions or substitutions of amino acid residues producing a silent change and resulting in functionally equivalent proteins, for identifying substances capable of interacting with a saposin-related polypeptide.

7. Use according to claim 6, wherein the fragments comprise cyclic peptides, retro-inverso-peptides or peptide mimetics having a length of at least four and up to 50 amino acids.

8. An in vitro method for differentiating or regenerating cells into functional pancreatic cells, the method comprising:
(a) cultivating cells capable of being differentiated or regenerated into pancreatic cells in the presence of an effective amount of a saposin-related protein in vitro
(b) allowing the cells to develop, to differentiate and/or to regenerate at least one pancreatic function; and
(c) optionally preparing an effective amount of the differentiated or regenerated pancreatic cells for transplantation into a patient in need thereof, particularly a human individual.

## Patentansprüche

1. Saposin-ähnliches Protein (Saposin-related protein) oder Nukleinsäure zur Verwendung in dem Schutz und/oder der Regeneration von Betazellen in Patienten, welche an Diabetes Typ-I oder Typ II leiden.

2. Saposin-ähnliches Protein oder Nukleinsäure zur Verwendung nach Anspruch 1 in Kombination mit einer anderen pharmazeutischen Zusammensetzung, welche nützlich zur Behandlung vom Betazell-Degeneration ist.

3. Saposin-ähnliches Protein oder Nukleinsäure zur Verwendung nach Anspruch 2, wobei die andere pharmazeutische Zusammensetzung Hormone, Wachstumsfaktoren oder immunmodulierende Mittel umfasst.

4. In vitro Verwendung von einem Saposin-ähnlichen Protein oder Nukleinsäure zum Stimulieren und/oder Induzieren der Differenzierung von Insulin produzierenden Zellen von Vorläuferzellen.

5. Verwendung nach Anspruch 4, wobei die Differenzierung von Vorläufer-, z.B. Stammzellen in Insulin produzierende Zellen in vitro umfasst:
(a) gegebenenfalls Aktivieren von einem oder mehreren Pankreasgenen in Vorläuferzellen,
(b) gegebenenfalls Aggregieren von den Zellen zum Bilden von embryoid bodies,
(c) Kultivieren von den Zellen oder embryoid bodies in spezifischen Differenzierungsmedien umfassend Saposin-ähnliches Proteinprodukt und
(d) Identifizieren und gegebenenfalls auswählen von Insulin produzierenden Zellen.

6. Verwendung eines Nukleinsäuremoleküls kodierend Saposin-ähnliches Polypeptid oder ein Homolog davon oder Verwendung eines Polypeptids kodierend dadurch, oder Verwendung eines Fragments des Nukleinsäuremoleküls oder des Polypeptids oder Verwendung von einer Polynukteotidsequenz, welche zum Hybridisieren von Nukleotidsequenzen kodierend Saposin-ähnliche Polypeptide oder einem Homolog davon fähig ist, oder Verwendung von Allelen von den Genen kodierend die Saposin-ähnliche Polypeptide, oder Verwendung von Nukleinsäuresequenzen kodierend die Polypeptide, wobei die Nukleinsäuresequenzen Deletionen, Insertionen oder Substitutionen von verschiedenen Nukleotiden tragen, welche in einem Polynukleotid kodierend das gleiche oder ein funktionell äquivalentes Polypeptid resultiert, oder Verwendung des kodierenden Polypeptids, wobei das Polypeptid Deletionen, Insertionen oder Substitutionen von Aminosäureresten umfasst, welches eine stille Änderung erzeugt und in funktionell äquivalenten Proteine resultiert, um Substanzen zu identifizieren, welche zum Interagieren mit einem Saposin-ähnlichen Polypeptid fähig sind.

7. Verwendung nach Anspruch 6, wobei die Fragmente zyklische Peptide, retro-inverso-Peptide oder Peptidmimetika umfassen, welche eine Länge von mindestens 4 und bis zu 50 Aminosäuren aufweisen.

8. In-vitro Verfahren zum Differenzieren oder Regenerieren von Zellen in funktionellen Pankreaszellen, wobei das Verfahren umfasst:
(a) Kultivieren von Zellen, welche fähig sind in Pankreaszellen in der Anwesenheit von einer effektiven Menge von einem Saposin-ähnlichen Protein in vitro, differenziert oder regeneriert zu werden,
(b) Erlauben der Zellen mindestens eine Pankreasfunktion zu entwickeln, zu differenzieren und/oder zu regenerieren, und
(c) gegebenenfalls herstellen eine effektive Menge von den differenzierten oder regenerierten Pankreaszellen für die Transplantation in einem bedürftigen Patienten, insbesondere einem menschlichen Individuum.

## Revendications

1. Protéine associée à la saposine ou acide nucléique destiné(e) à être utilisé(e) dans la protection et/ou la régénération de cellules bêta chez les patients souffrant de diabète de type I ou de type II.

2. Protéine associée à la saposine ou acide nucléique destiné(e) à être utilisé(e), selon la revendication 1, en combinaison avec une autre composition pharmaceutique utile pour traiter la dégénérescence des cellules bêta.

3. Protéine associée à la saposine ou acide nucléique destiné(e) à être utilisé(e), selon la revendication 2, dans laquelle ladite autre composition pharmaceutique comprend des hormones, des facteurs de croissance ou des agents de modulation immunitaires.

4. Utilisation in vitro d'une protéine associée à la saposine ou d'un acide nucléique pour stimuler et/ou induire la différentiation des cellules de production d'insuline des cellules progénitrices.

5. Utilisation selon la revendication 4, dans laquelle la différentiation des cellules progénitrices, e.g. des cellules souches en cellules productrices d'insuline in vitro comprend:
(a) éventuellement l'activation d'un ou de plusieurs gènes pancréatiques dans les cellules progénitrices,
(b) éventuellement l'agrégation desdites cellules pour former des corps embryoïdes,
(c) la culture desdites cellules ou de corps embryoïdes dans un milieu de différentiation spécifique contenant le produit de la protéine associée à la saposine et
(d) l'identification et éventuellement la sélection de cellules de production d'insuline.

6. Utilisation d'une molécule d'acide nucléique qui code pour le polypeptide associé à la saposine ou un de ses homologues ou utilisation d'un polypeptide codé par celle-ci, ou utilisation d'un fragment de ladite molécule d'acide nucléique ou dudit polypeptide ou utilisation d'une séquence de polynucléotides capables de s'hybrider à des séquences de nucléotides qui codent lesdits polypeptides ou un homologue de celles-ci, ou utilisation d'allèles des gènes codant lesdits polypeptides, dans lesquels lesdites séquences d'acides nucléiques portent des délétions, des insertions ou des substitutions de différents nucléotides résultant en un polynucléotide codant le même polypeptide ou un polypeptide fonctionnellement équivalent, ou utilisation dudit polypeptide codé, dans lequel le polypeptide contient des délétions, des insertions ou des substitutions de résidus d'acides aminés produisant un changement muet et ayant pour résultat des protéines fonctionnellement équivalentes pour l'identification de substances capables d'interagir avec le polypeptide associé à la saposine.

7. Utilisation selon la revendication 6, dans laquelle les fragments comprennent des peptides cycliques, des peptides rétro-inverso ou des peptides mimétiques ayant une longueur d'au moins 4 acides aminés et jusquà 50 acides aminés.

8. Méthode in vitro pour la différentiation ou la régénération de cellules en cellules fonctionnelles pancréatiques, la méthode comprenant:
a) la culture de cellules capables d'être différenciées et régénérées en cellules pancréatiques en présence d'une quantité efficace de protéines associées à la saposine in vitro,
b) la possibilité pour les cellules de développer, de différencier et/ou de régénérer au moins une fonction pancréatique et
c) éventuellement la préparation d'une quantité efficace de cellules pancréatiques différenciées ou régénérées pour la transplantation chez un patient en ayant besoin, particulièrement un individu humain.
